(19) Europäisches Patentamt European Patent Office Office européen des brevets

(11) **EP 4 437 940 B1**

(12) **EUROPEAN PATENT SPECIFICATION**

(45) Date of publication and mention
of the grant of the patent:
**06.05.2026  Bulletin 2026/19**

(21) Application number: **23864540.2**

(22) Date of filing: **17.08.2023**

(51) International Patent Classification (IPC):
*A61B 5/00* $^{(2006.01)}$     *A61B 5/318* $^{(2021.01)}$

(52) Cooperative Patent Classification (CPC):
**A61B 5/346; A61B 5/349; A61B 5/7221**

(86) International application number:
**PCT/CN2023/113618**

(87) International publication number:
**WO 2024/055807 (21.03.2024 Gazette 2024/12)**

(54) **METHOD AND DEVICE FOR EVALUATING ELECTROCARDIOSIGNAL QUALITY**

VERFAHREN UND VORRICHTUNG ZUR BEURTEILUNG DER QUALITÄT VON
ELEKTROKARDIOSIGNALEN

PROCÉDÉ ET DISPOSITIF D'ÉVALUATION DE LA QUALITÉ D'UN SIGNAL
D'ÉLECTROCARDIOGRAPHIE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority:  **15.09.2022   CN 202211120062**

(43) Date of publication of application:
**02.10.2024  Bulletin 2024/40**

(73) Proprietor: **Honor Device Co., Ltd.
Shenzhen, Guangdong 518040 (CN)**

(72) Inventor: **YIN, Haibo
Shenzhen, Guangdong 518040 (CN)**

(74) Representative: **Isarpatent
Patent- und Rechtsanwälte
Barth Hassa Peckmann & Partner mbB
Friedrichstraße 31
80801 München (DE)**

(56) References cited:
| | |
|---|---|
| CN-A- 105 997 054 | CN-A- 109 077 715 |
| CN-A- 110 226 919 | CN-A- 110 384 482 |
| CN-A- 112 971 797 | US-A- 5 842 997 |
| US-A1- 2012 016 249 | |

## Description

### TECHNICAL FIELD

This disclosure relates to the field of signal evaluation technologies, and in particular, the invention relates to an electrocardiogram signal quality evaluation method and an electronic device.

### BACKGROUND

An electrocardiogram signal is a weak signal of non-linearity, nonstationarity, and randomness, usually with a maximum magnitude of mV. Therefore, electrocardiogram signal monitoring usually requires an electrocardiogram monitoring apparatus to be closely attached to a surface of a living body to collect an electrocardiogram signal of the living body. It is clear that, due to features of the electrocardiogram signal and a scenario of the electrocardiogram signal monitoring, the electrocardiogram signal is highly susceptible to interference from within the living body (for example, electromyographic interference and respiratory interference) or outside the living body (for example, power frequency interference and a signal pickup process).

At present, a collected electrocardiogram signal may be accompanied by a significant number of noise or abnormal signals (resulting from an abnormal acquisition process), resulting in the electrocardiogram signal being swamped with the noise and/or abnormal signals and losing some original features. In particular, when the electrocardiogram signal has some pathological features, these pathological features may be disturbed by the noise and/or abnormal signals, resulting in a reduced accuracy of an early warning algorithm associated with a pathological signal. US 2012/016249 A1 relates to a method and system for assessing the quality of ECG signals by analyzing a variety of signal features and classifying the signals accordingly. US 5,842,997 A relates to a method and apparatus for identifying artifact-contaminated ECG data segments.

### SUMMARY

In view of this, an object of the present invention is to provides an electrocardiogram signal quality evaluation method and an electronic device which can detect a normal signal in an electrocardiogram signal. This object is solved by the attached independent claims and further embodiments and improvements of the invention are listed in the attached dependent claims. Hereinafter, up to the "brief description of the drawings", expressions like "...aspect according to the invention", "according to the invention", or "the present invention", relate to technical teaching of the broadest embodiment as claimed with the independent claims. Expressions like "implementation", "design", "optionally", "preferably", "scenario", "aspect" or similar relate to further embodiments as claimed, and expressions like "example", "...aspect according to an example", "the disclosure describes", or "the disclosure" describe technical teaching which relates to the understanding of the invention or its embodiments, which, however, is not claimed as such.

To achieve the foregoing objective, the following technical solutions are used in this application:

According to a first aspect according to the invention, this disclosure provides an electrocardiogram signal quality evaluation method. The method includes:

obtaining a first electrocardiogram signal; and
calculating a peak variability feature and a peak number variability feature of the first electrocardiogram signal, where if the peak variability feature of the first electrocardiogram signal is less than a first threshold, and the peak number variability feature of the first electrocardiogram signal is less than a second threshold, the first electrocardiogram signal is a normal signal, where the first threshold represents an abnormal dividing point of the peak variability feature, the first threshold is determined based on peak variability features of a plurality of electrocardiogram signal samples, the second threshold represents an abnormal dividing point of the peak number variability feature, and the second threshold is determined based on the peak number variability features of the plurality of electrocardiogram signal samples.

In this disclosure, the peak variability feature and the peak number variability features of the plurality of electrocardiogram signal samples are counted, based on the peak variability feature of the plurality of electrocardiogram signal samples as one abnormal dividing point, the peak number variability features of the plurality of electrocardiogram signal samples is used as another abnormal dividing point; and a type of a to-be-evaluated electrocardiogram signal is determined based on a relationship between the peak variability feature and the peak number variability feature of the to-be-evaluated electrocardiogram signal and the two abnormal dividing points. A normal signal may be accurately distinguished by this method.

In an implementation of the first aspect, obtaining a first electrocardiogram signal includes:

obtaining an original electrocardiogram signal with first duration; and
performing filtering processing on the original electrocardiogram signal to obtain the first electrocardiogram signal.

In this disclosure, variability features associated with peaks are used as different types of features of the electrocardiogram signal. Therefore, to improve the accuracy of the feature, filtering processing is required to remove glitches in a waveform of the electrocardiogram signal.

In another implementation of the first aspect, performing filtering processing on the original electrocardiogram signal includes:
performing low-pass filtering processing and high-pass filtering processing on the original electrocardiogram signal.

In another implementation of the first aspect, calculating a peak variability feature and a peak number variability feature of the first electrocardiogram signal includes:

performing segmentation processing on the first electrocardiogram signal to obtain a plurality of signal segments;
calculating a peak list and a peak number of each signal segment, where the peak list includes N peaks, and N represents the peak number;
obtaining the peak variability feature of the first electrocardiogram signal based on peak lists of the plurality of signal segments; and
obtaining the peak number variability feature of the first electrocardiogram signal based on peak numbers of the plurality of signal segments.

In embodiments of this disclosure, segmentation processing is performed on the first electrocardiogram signal to obtain a plurality of signal segments, the peak variability feature of the first electrocardiogram signal is determined based on a data feature of peak lists of the plurality of signal segments, and the peak number variability feature of the first electrocardiogram signal is determined based on a data feature of the peak number of the plurality of signal segments.

In another implementation of the first aspect, the signal segment includes a plurality of discrete data points, and calculating a peak list of each signal segment includes:
calculating an absolute value of a difference between each two adjacent data points in a first signal segment, where the first signal segment is any signal segment in the signal segments, a peak list of the first signal segment includes one or more first peaks, the first peak is an absolute value of a difference that is greater than an absolute value of a previous difference, greater than an absolute value of a next difference, and greater than a peak searching threshold, and the peak searching threshold is determined based on absolute values of differences respectively corresponding to the plurality of signal segments of the first electrocardiogram signal.

In another implementation of the first aspect, a method for determining the peak searching threshold includes:

calculating a first product of a first constant and a first percentile in a set of the absolute values of the differences corresponding to the plurality of signal segments of the first electrocardiogram signal;
calculating a second product of a second constant and a second percentile in the set of the absolute values of the differences corresponding to the plurality of signal segments of the first electrocardiogram signal; and
calculating a difference between the first product and the second product to obtain the peak searching threshold.

In another implementation of the first aspect, obtaining the peak variability feature of the first electrocardiogram signal based on peak lists of the plurality of signal segments includes:

calculating a standard deviation and a mean value of peaks of the plurality of signal segments of the first electrocardiogram signal; and
calculating a ratio of the standard deviation to the mean value of the peaks of the plurality of signal segments to obtain the peak variability feature of the first electrocardiogram signal.

In another implementation of the first aspect, obtaining the peak number variability feature of the first electrocardiogram signal based on peak numbers of the plurality of signal segments includes:

calculating a first difference between a largest value and a smallest value of the peak numbers of the plurality of signal segments of the first electrocardiogram signal;
calculating a median value of the peak numbers of the plurality of signal segments of the first electrocardiogram signal; and
calculating a ratio of the first difference value to the median value to obtain the peak number variability feature of the first electrocardiogram signal.

In another implementation of the first aspect, a manner of determining the first threshold and the second threshold includes:

obtaining a plurality of electrocardiogram signal samples, where duration of each of the plurality of electrocardiogram signal samples is the first duration;
calculating a peak variability feature and a peak number variability feature of each electrocardiogram signal sample;
obtaining the first threshold based on peak variability features of the plurality of electrocardiogram signal samples; and
obtaining the second threshold based on peak number variability features of the plurality of electrocardiogram signal samples.

In another implementation of the first aspect, obtaining the first threshold based on a peak variability feature of the plurality of electrocardiogram signal samples includes:

calculating a third product of a third constant and a third percentile in the peak variability feature of the plurality of electrocardiogram signal samples;
calculating a fourth product of a fourth constant and a fourth percentile in the peak variability feature of the plurality of electrocardiogram signal samples; and
calculating a difference between the third product and the fourth product to obtain the first threshold.

In another implementation of the first aspect, obtaining the second threshold based on peak number variability features of the plurality of electrocardiogram signal samples includes:

calculating a fifth product of a fifth constant and a fifth percentile in the peak number variability features of the plurality of electrocardiogram signal samples;
calculating a sixth product of a sixth constant and a sixth percentile in the peak number variability features of the plurality of electrocardiogram signal samples; and
calculating a difference between the fifth product and the sixth product to obtain the second threshold.

In another implementation of the first aspect, the method further includes:

if a peak variability feature of the first electrocardiogram signal is greater than a first threshold, and a peak number variability feature of the first electrocardiogram signal is greater than a second threshold, the first electrocardiogram signal is an abnormal signal;
if the peak variability feature of the first electrocardiogram signal is greater than or equal to the first threshold, and the peak number variability feature of the first electrocardiogram signal is less than or equal to the second threshold, the first electrocardiogram signal is a noise signal; or
if the peak variability feature of the first electrocardiogram signal is less than or equal to the first threshold, and the peak number variability feature of the first electrocardiogram signal is greater than or equal to the second threshold, the first electrocardiogram signal is a noise signal.

In another implementation of the first aspect, obtaining a plurality of electrocardiogram signal samples includes:

obtaining a plurality of original electrocardiogram signals;
performing data segmentation on the plurality of original electrocardiogram signals to obtain a plurality of original electrocardiogram signals with the first duration; and
performing low-pass filtering processing and high-pass filtering processing on original electrocardiogram signals with the first duration to obtain the plurality of electrocardiogram signal samples.

In another implementation of the first aspect, a manner of calculating a peak variability feature of each electrocardiogram signal sample is the same as a manner of calculating the peak variability feature of the first electrocardiogram signal; and
a manner of calculating the peak number variability feature of each electrocardiogram signal sample is the same as a manner of calculating the peak number variability feature of the first electrocardiogram signal.

According to a second aspect according to the invention, an electronic device is provided, including a processor. The processor is configured to run a computer program stored in a memory, to implement steps of the method according to any one of the first aspect of this application.

According to a third aspect not claimed, a chip system is provided, including a processor. The processor is coupled to a memory and executes a computer program stored in a memory, to implement steps of the method according to any one of the first aspect of this application.

According to a fourth aspect not claimed, a computer-readable storage medium is provided. The computer-readable storage medium stores a computer program, and the computer program, when executed by one or more processors, implements steps of the method according to any one of the first aspect of this application.

According to a fifth aspect not claimed, this application provides a computer program product, and the computer program product, when run on a device, causes the device to perform the steps of the method according to any one of the first aspect of this application.

It may be understood that, for beneficial effects of the second aspect to the fifth aspect, reference may be made to the relevant description in the first aspect, and details are not described herein again.

## BRIEF DESCRIPTION OF DRAWINGS

FIG. 1 is a schematic diagram of a hardware structure of an electronic device according to an embodiment of this application;

FIG. 2 is a schematic flowchart of an electrocardiogram signal quality evaluation method according to an embodiment of this application;

FIG. 3 is a schematic flowchart of an electrocardiogram signal feature learning process according to an embodiment of this application;

FIG. 4 is a schematic waveform diagram of an electrocardiogram signal before filtering according to an embodiment of this application;

FIG. 5 is a schematic waveform diagram of an electrocardiogram signal obtained through filtering according to an embodiment of this application;

FIG. 6 is a schematic diagram of obtained peak points according to an embodiment of this application;

FIG. 7 is a schematic flowchart of an electrocardiogram signal quality evaluation process according to an embodiment of this application;

FIG. 8 is a schematic waveform diagram of four signal segments before filtering, after filtering, and at peak points according to an embodiment of this application; and

FIG. 9 is a classification result of four electrocardiogram signals and more signal segments according to an embodiment of this application.

## DESCRIPTION OF EMBODIMENTS

In the following description, for the purpose of illustration rather than limitation, specific details such as the specific system structure and technology are proposed to thoroughly understand embodiments of this application. However, a person skilled in the art should know that this application may be practiced in other embodiments without these specific details.

It should be understood that when used in this specification of this application and the appended claims, the term "comprise" indicates the presence of described features, wholes, steps, operations, elements and/or components, but do not exclude the presence or addition of one or more other features, wholes, steps, operations, elements, components and/or a set thereof.

It should be further understood that in embodiments of this application, "one or more" refers to one, two, or two or more. "And/or" describes an association relationship between associated objects and represents that three relationships may exist. For example, A and/or B may represent: Only A exists, both A and B exist, and only B exists, where A and B may be singular or plural. The character "/" generally represents an "or" relationship between the associated objects.

In addition, descriptions in this specification of this application and the appended claims, terms such as "first", "second", "third", and "fourth" are used only to distinguish descriptions, and should not be understood as indicating or implying relative importance.

"One embodiment" or "some embodiments" described in this specification of this application means that specific features, structures, or characteristics described with reference to the embodiment are included in one or more embodiments of this application. Therefore, the statements "in one embodiment", "in some embodiments", "in some other embodiments", "in still some other embodiments" appearing at different positions in this specification do not necessarily refer to the same embodiment, but mean "one or more but not all embodiments", unless otherwise specially emphasized in other ways. The terms "comprise", "include", "have", and variations thereof all mean "include but is not limited to", unless otherwise specially emphasized in other ways.

An electrocardiogram signal quality evaluation method provided in embodiments of this application may be used in electronic devices such as an electrocardiogram monitoring device, a wearable device, and the like. It is clear that, the method may alternatively be used in electronic devices such as a mobile phone connected to an electrocardiogram monitoring apparatus. A specific type of the electronic device is not limited in embodiments of this application.

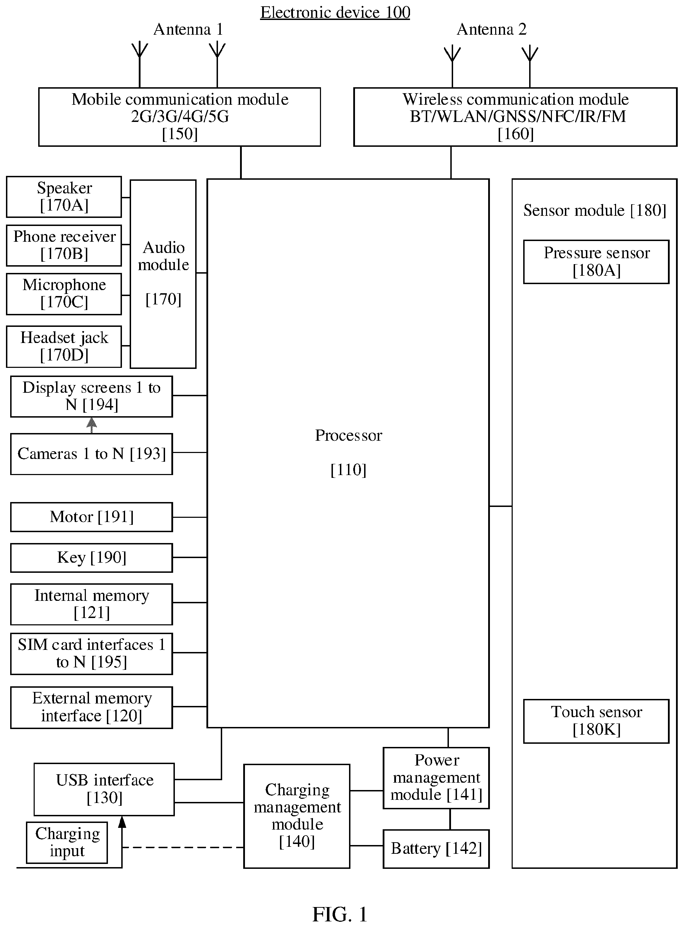

FIG. 1 is a schematic structural diagram of an electronic device. The electronic device 100 may include a processor 110, an external memory interface 120, an internal memory 121, a universal serial bus (universal serial bus, USB) interface 130, a charging management module 140, a power management module 141, a battery 142, an antenna 1, an antenna 2, a mobile communication module 150, a wireless communication module 160, an audio module 170, a speaker 170A, a phone receiver 170B, a microphone 170C, a headset jack 170D, a sensor module 180, a key 190, a motor 191, a camera 193, a display screen 194, a subscriber identification module (subscriber identification module, SIM) card interface 195, and the like. The sensor module 180 may include a pressure sensor 180A, a touch sensor 170B, and the like.

It may be understood that an example structure in this embodiment of this application does not constitute a specific limitation on the electronic device 100. In some other embodiments of this application, the electronic device 100 may include more or fewer components than those shown in the figure, or some components may be combined, or some components may be split, or components are arranged in different manners. The components in the figure may be implemented by hardware, software, or a combination of software and hardware.

The processor 110 may include one or more processing units. For example: the processor 110 may include an application processor (application processor, AP), a modem processor, a graphics processing unit (graphics processing unit, GPU), an image signal processor (image signal processor, ISP), a controller, a memory, a video codec, a digital signal processor (digital signal processor, DSP), a baseband processor, and/or a neural-network processing unit (neural-network processing unit, NPU). Different processing units may be independent components, or may be integrated into one or more processors.

The controller may be a nerve center and a command center of the electronic device 100. The controller may generate an operation control signal based on an instruction operation code and a timing signal, to complete control of fetching instructions and executing the instructions.

A memory may further be arranged in the processor 110, and is configured to store instructions and data. In some embodiments, the memory in the processor 110 is a cache memory. The memory may store instructions or data that are/is recently used or cyclically used by the processor 110. If the processor 110 needs to use the instructions or the data again, the processor 110 may directly invoke the instructions or the data from the memory, repeated access is avoided, and a waiting time of the processor 110 is reduced, thereby improving system efficiency.

The USB interface 130 is an interface that complies with the USB standard specification, and may be specifically a Mini USB interface, a Micro USB interface, a USB Type C interface, and the like. The USB interface 130 may be configured to connect to the charger to charge the electronic device 100, or may be used for data transmission between the electronic device 100 and a peripheral device.

The external memory interface 120 may be configured to connect to an external storage card such as a Micro SD card, to expand a storage capability of the electronic device 100. The external storage card communicates with the processor 110 by using the external memory interface 120, to implement a data storage function, for example, store files such as music or a video in the external storage card.

The internal memory 121 may be configured to store computer-executable program code, and the executable program code includes instructions. The processor 110 runs instructions stored in the internal memory 121, to perform various function applications and data processing of the electronic device 100. The internal memory 121 may include a program storage region and a data storage region. The program storage region may store an operating system, an application required by at least one function (for example, a sound playing function or an image playing function), and the like.

In addition, the internal memory 121 may include a high-speed random access memory, and may also include a non-volatile memory, for example, at least one magnetic disk memory, a flash storage device, or a universal flash storage (universal flash storage, UFS).

The charging management module 140 is configured to receive a charging input from a charger. The charger may be a wireless charger, or may be a wired charger. In some embodiments of wired charging, the charging management module 140 may receive a charging input from the wired charger through the USB interface 130.

The power management module 141 is configured to connect the battery 142, the charging management module 140, and the processor 110. The power management module 141 receives an input of the battery 142 and/or the charging management module 140, to supply power to the processor 110, the internal memory 121, the external memory, the display screen 194, the camera 193, the wireless communication module 160, and the like.

In some other embodiments, the power management module 141 may alternatively be arranged in the processor 110. In some other embodiments, the power management module 141 and the charging management module 140 may alternatively be arranged in a same device.

A wireless communication function of the electronic device 100 may be implemented through the antenna 1, the antenna 2, the mobile communication module 150, the wireless communication module 160, the modem processor, the baseband processor, and the like.

The antenna 1 and the antenna 2 are configured to transmit and receive an electromagnetic wave signal. Each antenna of the electronic device 100 may be configured to cover one or more communication frequency bands. Different antennas may further be reused to improve utilization of the antennas. For example: the antenna 1 may be reused as a diversity antenna of a wireless local area network. In some other embodiments, the antenna may be used in combination with a tuning switch.

The mobile communication module 150 may provide a solution to wireless communication such as 2G/3G/4G/5G used in the electronic device 100. The mobile communication module 150 may include at least one filter, a switch, a power amplifier, a low noise amplifier (low noise amplifier, LNA), or the like. The mobile communication module 150 may receive an electromagnetic wave through the antenna 1, perform processing, such as filtering and amplification, on the received electromagnetic wave, and transmit the processed electromagnetic wave to the modem processor for demodulation. The mobile communication module 150 may further amplify a signal modulated by the modem processor, and convert the signal into an electromagnetic wave for radiation through the antenna 1.

The wireless communication module 160 may provide a solution to wireless communication used in the electronic device 100, for example, a wireless local area network (wireless local area networks, WLAN) (for example, a wireless fidelity (wireless fidelity, Wi-Fi) network), Bluetooth (bluetooth, BT), a global navigation satellite system (global navigation satellite system, GNSS), frequency modulation (frequency modulation, FM), near field communication (near field communication, NFC), and an infrared (infrared, IR) technology. The wireless communication module 160 may be one or more devices into which at least one communication processing module is integrated. The wireless communication module 160 receives an electromagnetic wave by using the antenna 2, performs frequency modulation and filtering processing on the electromagnetic wave signal, and sends a processed signal to the processor 110. The wireless communication module 160 may alternatively receive a to-be-sent signal from the processor 110, and perform frequency modulation and amplification on the signal, and convert the signal into an electromagnetic wave for radiation by using the antenna 2.

In some embodiments, in the electronic device 100, the antenna 1 and the mobile communication module 150 are coupled, and the antenna 2 and the wireless communication module 160 are coupled, so that the electronic device 100 can communicate with a network and another device by using a wireless communication technology.

The electronic device 100 may implement an audio function by using an audio module 170, a speaker 170A, a phone receiver 170B, a microphone 170C, a headset jack 170D, an application processor, and the like, for example, music playing or recording.

The audio module 170 is configured to convert a digital audio signal into an analog audio signal to output, and is further configured to convert an analog audio input into a digital audio signal. The audio module 170 may alternatively be configured to encode and decode an audio signal. In some embodiments, the audio module 170 may be arranged in the processor 110, or some functional modules of the audio module 170 are arranged in the processor 110.

The speaker 170A, also referred to as a "speaker", is configured to convert an audio electrical signal into a sound signal. Music can be listened to or a hands-free call may be answered by using the speaker 170A in the electronic device 100.

The phone receiver 170B, also referred to as a "receiver", is configured to convert an audio electrical signal into a sound signal. When the electronic device 100 is configured to answer a call or receive voice information, the phone receiver 170B may be put close to a human ear, to receive a voice.

The microphone 170C, also referred to as a "mouthpiece" or a "megaphone", is configured to convert a sound signal into an electrical signal. When making a call or sending voice information, a user may speak with the mouth approaching the microphone 170C, to input a sound signal into the microphone 170C. At least one microphone 170C may be arranged in the electronic device 100. In some other embodiments, two microphones 170C may be arranged in the electronic device 100, to monitor voice information and implement a noise reduction function. In some other embodiments, three, four, or more microphones 170C may be alternatively arranged in the electronic device 100, to collect a sound signal, implement noise reduction, recognize a sound source, implement a directional recording function, and the like.

The headset jack 170D is configured to connect a wired headset. The headset jack 170D may be a USB interface 130, or may be a 3.5 mm open mobile terminal platform (open mobile terminal platform, OMTP) standard interface or cellular telecommunications industry association of the USA (cellular telecommunications industry association of the USA, CTIA) standard interface.

The pressure sensor 180A is configured to sense a pressure signal, and may convert the pressure signal into an electrical signal. In some embodiments, the pressure sensor 180A may be arranged on the display screen 194. There are a plurality of types of pressure sensors 180A, for example, a resistive pressure sensor, an inductive pressure sensor, and a capacitive pressure sensor. The capacitive pressure sensor may include at least two parallel plates having conductive materials. When force is exerted on the pressure sensor 180A, capacitance between electrodes changes. The electronic device 100 determines pressure strength based on a change in the capacitance. When a touch operation is performed on the display screen 194, the electronic device 100 detects strength of the touch operation by using the pressure sensor 180A. The electronic device 100 may also calculate a touch position based on a detection signal of the pressure sensor 180A.

The touch sensor 180K is also referred to as a "touch panel". The touch sensor 180K may be arranged on the display screen 194, and the touch sensor 180K and the display screen 194 form a touchscreen, which is also referred to as a "touch screen". The touch sensor 180K is configured to detect a touch operation performed on or near the touch sensor 180K. The touch sensor may transmit the detected touch operation to the application processor, to determine a touch event type. The touch sensor 180K may provide a visual output related to the touch operation by using the display screen 194. In some other embodiments, the touch sensor 180K may alternatively be arranged on a surface of the electronic device 100, and is located on a position different from that of the display screen 194.

The key 190 includes a power key, a volume key, or the like. The key 190 may be a mechanical key, or may be a touch key. The electronic device 100 may receive a key input, and generate a key signal input related to user setting and function control of the electronic device 100.

The motor 191 may generate a vibration prompt. The motor 191 may be configured to provide a vibration prompt for an incoming call, and may be further configured to provide a touch vibration feedback.

The electronic device 100 implements a display function by using the GPU, the display screen 194, the application processor, and the like. The GPU is a microprocessor for image processing, and is connected to the display screen 194 and the application processor. The GPU is configured to perform mathematical and geometric calculation for graphics rendering. The processor 110 may include one or more GPUs that execute program instructions to generate or change display information.

The display screen 194 is configured to display an image, a video, or the like. In some embodiments, the electronic device 100 may include 1 or N display screens 194, and N is a positive integer greater than 1.

The camera 193 is configured to capture a static image or a video. In some embodiments, the electronic device 100 may include one or N cameras 193, and N is a positive integer greater than 1.

The SIM card interface 195 is configured to connect a SIM card. The SIM card may be inserted into the SIM card interface 195 or plugged from the SIM card interface 195, to come into contact with or be separated from the electronic device 100. The electronic device 100 may support one or N SIM card interfaces, and N is a positive integer greater than 1.

A specific structure of an execution body of the electrocardiogram signal quality evaluation method is not specifically limited in embodiments of this application, provided that processing can be performed based on the electrocardiogram signal quality evaluation method provided in embodiments of this application by running code that records the electrocardiogram signal quality evaluation method provided in embodiments of this application. For example, the electrocardiogram signal quality evaluation method provided in embodiments of this application may be performed by a functional module in an electronic device that can invoke a program and execute the program, or a processing apparatus, for example, a chip, used in an electronic device.

An electrocardiogram signal is a weak signal of non-linearity, nonstationarity, and randomness, usually with a maximum magnitude of mV. Therefore, electrocardiogram signal monitoring usually requires an electrocardiogram monitoring apparatus to be closely attached to a surface of a living body to collect an electrocardiogram signal of the living body. It is clear that, due to features of the electrocardiogram signal and a scenario of the electrocardiogram signal monitoring, the electrocardiogram signal is highly susceptible to interference from within the living body (for example, electromyographic interference and respiratory interference) or outside the living body (for example, power frequency interference and a signal pickup process). Consequently, a collected electrocardiogram signal may be accompanied by a significant number of noise or abnormal signals (resulting from an abnormal acquisition process), resulting in the electrocardiogram signal being swamped with the noise and/or abnormal signals and losing some original features. In particular, when the electrocardiogram signal has some pathological features, these pathological features may be disturbed by the noise and/or abnormal signals, resulting in a reduced accuracy of an early warning algorithm associated with a pathological signal.

Therefore, embodiments of this application provide the electrocardiogram signal quality evaluation method that can detect a normal signal in the electrocardiogram signal, so that early warning of pathology can be performed based on the detected normal signal.

The electrocardiogram signal quality evaluation algorithm provided in embodiments of this application may be used in an electronic device used for electrocardiogram monitoring. Because a wearable electrocardiogram monitoring device needs to be worn on a living body when monitoring the electrocardiogram signal, the wearable electrocardiogram monitoring device may be worn less normatively or may not fully meet a requirement of electrocardiogram monitoring. The electrocardiogram signal monitored by the wearable electrocardiogram monitoring device is more likely to be contaminated by the noise or abnormal signal. Therefore, embodiments of this application are more suitable for the wearable electrocardiogram monitoring device.

The electrocardiogram signal quality evaluation method provided in embodiments of this application uses a peak variability feature and a peak number variability feature of the electrocardiogram signal as features for evaluating whether the electrocardiogram signal is a normal signal, an abnormal signal, or a noise signal.

In embodiments of this application, the normal signal may or may not include a pathological feature, depending on a physical condition of the living body. The abnormal signal is an electrocardiogram signal collected in case of an abnormality in an acquisition process caused by an error in the user wearing the wearable electrocardiogram monitoring device; and the noise signal is a variety of noise in a test process.

It should be noted that, in embodiments of this application, when a final evaluation shows that the electrocardiogram signal is a normal signal, it does not represent that the normal signal does not include an abnormal point or a noise point. However, the included abnormal point and/or noise point are/is not enough to affect subsequent processing of the signal. Similarly, when the final evaluation shows that the electrocardiogram signal is a noise signal, it does not represent that the noise signal does not include a normal point or an abnormal point. However, there are fewer included normal points and/or abnormal points, and most points are noise points. Similarly, when the final evaluation shows that the electrocardiogram signal is an abnormal signal, it does not represent that the abnormal signal does not include a normal point or a noise point. However, there are fewer included normal points and/or abnormal points, and most points are abnormal points.

In a specific implementation, an abnormal dividing point of the peak variability feature and an abnormal dividing point of the peak number variability feature need to be set. Whether the electrocardiogram signal is the normal signal, the abnormal signal, or the noise signal is determined based on a relationship between the peak variability feature of the electrocardiogram signal and the abnormal dividing point of the peak variability feature, and a relationship between the peak number variability feature of the electrocardiogram signal and the abnormal dividing point of the peak number variability feature.

To ensure that the electronic device, after monitoring the electrocardiogram signal, can determine a quality (normal, abnormal, or noise) of the monitored electrocardiogram signal based on the relationship between the peak variability feature of the monitored electrocardiogram signal and the abnormal dividing point of the peak variability feature, and the relationship between the peak number variability feature of the monitored electrocardiogram signal and the abnormal dividing point of the peak number variability feature, the abnormal dividing point of the peak variability feature and the abnormal dividing point of the peak number variability feature need to be predetermined.

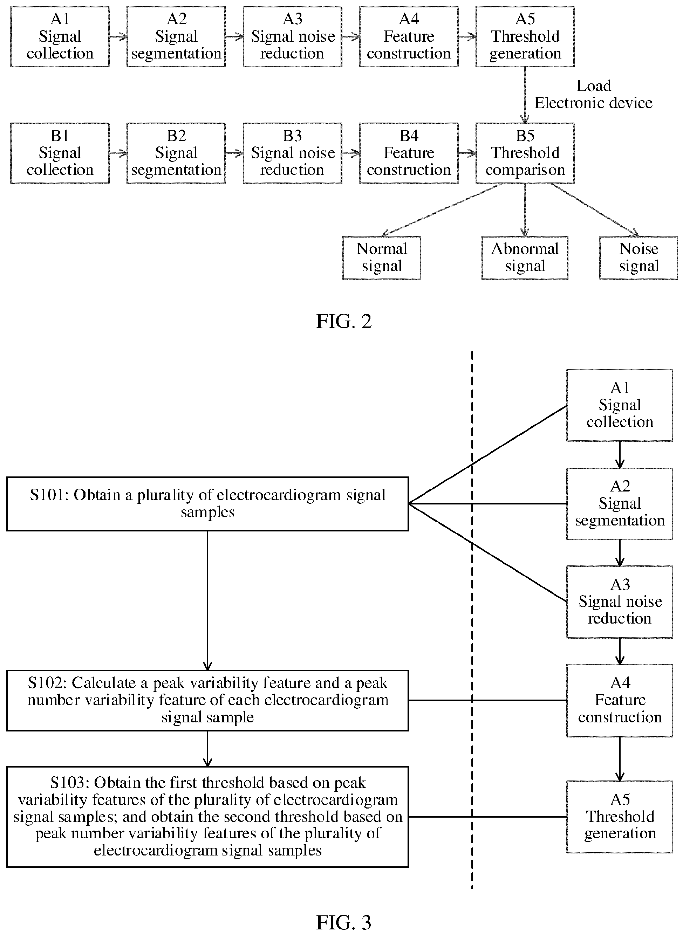

Refer to FIG. 2. Step A1 to step A5 in FIG. 2 are an example of determining the abnormal dividing point of the peak variability feature (which may be denoted as a first threshold) and the abnormal dividing point of the peak number variability feature (which may be denoted as a second threshold).

Step A1: Signal collection.

In this embodiment of this application, the abnormal dividing point for distinguishing between different types of electrocardiogram signals needs to be learned. When the abnormal dividing point is learned, a plurality of multi-user and multi-scene original electrocardiogram signals need to be collected. It is clear that, in an actual application, a plurality of original electrocardiogram signals may be obtained from an original electrocardiogram signal sample library, and a manner in which the original electrocardiogram signal is obtained is not limited in this embodiment of this application.

Step A2: Signal segmentation.

In a specific implementation, signal segmentation in time domain performed on the plurality of original electrocardiogram signals of multiple-user and multiplescene is required, and finally a plurality of original electrocardiogram signals of fixed duration are obtained, to learn the abnormal dividing point from the plurality of original electrocardiogram signals of fixed duration.

Step A3: Signal noise reduction.

Before the plurality of original electrocardiogram signals of fixed duration are learned, filtering processing needs to be performed on the plurality of original electrocardiogram signals of fixed duration to eliminate glitches. After filtering processing is performed, a plurality of electrocardiogram signal samples are obtained.

Step A4: Feature construction.

In this embodiment of this application, peak variability features and peak number variability features of the plurality of electrocardiogram signal samples are used as features to distinguish between different types of signals.

Therefore, feature construction needs to be performed on each electrocardiogram signal sample to obtain the peak variability feature and the peak number variability feature of each electrocardiogram signal sample.

Step A5: Threshold generation.

The abnormal dividing point of the peak variability feature, namely, the first threshold, is determined by counting the peak variability features of the plurality of electrocardiogram signal samples.

The abnormal dividing point of the peak variability feature, namely, the second threshold, is determined by counting the peak number variability features of the plurality of electrocardiogram signal samples.

Through step A1 to step A5, the abnormal dividing points of the two features, namely, the first threshold and the second threshold, can be determined based on the plurality of electrocardiogram signal samples.

After the first threshold and the second threshold are determined, the first threshold, the second threshold, and an algorithm for generating the peak variability feature and the peak number variability feature may be preset into an electronic device for electrocardiogram signal monitoring. The electronic device can perform quality evaluation on the electrocardiogram signal monitored by the electronic device by using the first threshold, the second threshold, and the algorithm for generating the peak variability feature and peak number variability feature. For details, refer to step B1 to step B5 in FIG. 2.

Step B1: Signal collection.

In this embodiment of this application, the electrocardiogram signal may be monitored in real time by using the electronic device.

Step B2: Signal segmentation.

In this embodiment of this application, a manner for performing signal segmentation on the electrocardiogram signal monitored in real time is the same as that in step A2, and the monitored electrocardiogram signal is segmented into electrocardiogram signals with fixed duration. The duration of the segmented electrocardiogram signal in step B2 is the same as the duration of the segmented electrocardiogram signal in step A2.

In an actual application, after the electronic device performs electrocardiogram signal monitoring on the living body, signal segmentation may be performed on the monitored original electrocardiogram signal to obtain the plurality of electrocardiogram signals of fixed duration.

Alternatively, during electrocardiogram signal monitoring of the living body by the electronic device, each time an electrocardiogram signal of fixed duration is collected, the electrocardiogram signal with the fixed duration may be segmented, and each time an electrocardiogram signal of fixed duration is obtained, subsequent processing is performed on the obtained electrocardiogram signal with the fixed duration.

A specific implementation is not limited in this embodiment of this application.

Step B3: Signal noise reduction.

In this step, a noise reduction manner for the segmented electrocardiogram signal with the fixed duration is the same as that in step A3.

Step B4: Feature construction.

In this step, a feature construction manner for the electrocardiogram signal with the fixed duration after noise reduction is the same as that in step A4.

Step B2 to step B4 are a process of calculating the peak variability feature and the peak number variability feature of the monitored electrocardiogram signal by using an algorithm preset in the electronic device for generating the peak variability feature and the peak number variability feature.

Step B5: Threshold comparison.

The peak variability feature and the peak signal variability feature of the monitored electrocardiogram signal may be obtained through step B1 to step B4, the obtained peak variability feature is compared with the first threshold preset in the electronic device, and the obtained peak number variability feature is compared with the second threshold preset in the electronic device.

In an example, if the peak variability feature of the monitored electrocardiogram signal (with the fixed duration) is greater than the first threshold, and the peak number variability feature of the monitored electrocardiogram signal is greater than the second threshold, the monitored electrocardiogram signal is the abnormal signal.

If the peak variability feature of the monitored electrocardiogram signal (with the fixed duration) is greater than or equal to the first threshold, and the peak number variability feature of the monitored electrocardiogram signal is less than or equal to the second threshold, the monitored electrocardiogram signal is the noise signal.

If the peak variability feature of the monitored electrocardiogram signal (with the fixed duration) is less than or equal to the first threshold, and the peak number variability feature of the monitored electrocardiogram signal is greater than or equal to the second threshold, the monitored electrocardiogram signal is the noise signal.

If the peak variability feature of the monitored electrocardiogram signal (with the fixed duration) is less than the first threshold, and the peak number variability feature of the monitored electrocardiogram signal is less than the second threshold, the monitored electrocardiogram signal is the normal signal.

In this embodiment of this application, it is found by analyzing a large number of electrocardiogram signals that: there are large differences between peaks and peak numbers of the normal signal, the abnormal signal, and the noise signal with the fixed duration. Therefore, in this embodiment of this application, the peak variability feature and the peak number variability feature of the electrocardiogram signal with the fixed duration are used as features for electrocardiogram signal classification. Then, the abnormal dividing point for electrocardiogram signal classification is obtained by learning the foregoing two features of the plurality of electrocardiogram signals with the fixed duration. Based on a relationship between the foregoing two features of the monitored electrocardiogram signal and the abnormal dividing point, a type of the monitored electrocardiogram signal is determined.

In addition, in the foregoing embodiment, if the first threshold, the second threshold, and the algorithm for calculating the two features are preset in an electronic device of a model A, the original electrocardiogram signal in step A1 may be acquired on an electronic device of the same model A. In this way, signal classification can be more accurate through a consistent electrocardiogram signal. It is clear that, after a first threshold and a second threshold are obtained from multi-user and multi-scene original electrocardiogram signals collected on an electronic device 1 of the model A, the first threshold, the second threshold, and the corresponding algorithm for calculating the two features may be preset on a plurality of electronic devices also of a type A, for example, an electronic device 2 of the type A, an electronic device 3 of the type A, and an electronic device 4 of the type A.

It is clear that, in an actual application, if an electronic device of another model (for example, a model B) has the same electrocardiogram monitoring apparatus and the same electrocardiogram monitoring manner as the electronic device of the present model (for example, the model A), the first threshold, the second threshold, and the corresponding algorithm for calculating the two features may be preset in the electronic device of the another model.

To have a clearer understanding of various steps of the foregoing embodiment, refer to the schematic flowchart shown in FIG. 3. The flowchart shown in FIG. 3 corresponds to step A1 to step A5 in the embodiment shown in FIG. 2.

S101: Obtain a plurality of electrocardiogram signal samples, where duration of each of the plurality of electrocardiogram signal samples is the first duration.

In this embodiment of this application, the plurality of electrocardiogram signal samples may be understood as a plurality of electrocardiogram signal samples with fixed duration that are obtained from step A1 to step A3 and on which filtering processing is performed.

In another embodiment of this application, the obtaining a plurality of electrocardiogram signal samples includes the following steps:

Step A1: Obtain a plurality of original electrocardiogram signals.

As described above, the plurality of original electrocardiogram signals may be multi-user and multi-scene original electrocardiogram signals collected by an electronic device of the same type (or having the same electrocardiogram monitoring apparatus and the same electrocardiogram monitoring manner).

Step A2: Perform data segmentation on the plurality of original electrocardiogram signals to obtain a plurality of original electrocardiogram signals with first duration.

After the plurality of original electrocardiogram signals are obtained, signal segmentation needs to be performed on each original electrocardiogram signal.

Because a process of signal segmentation is used to obtain the plurality of original electrocardiogram signals with the fixed duration, an original electrocardiogram signal whose total duration is shorter than the fixed duration is discarded. One or more original electrocardiogram signals with fixed duration are segmented from each original electrocardiogram signal whose total duration is longer than or equal to the fixed duration, and the fixed duration may be the first duration.

It should be noted that, an original electrocardiogram signal with the first duration may be segmented in each original electrocardiogram signal whose total duration is longer than or equal to the first duration.

Alternatively, as many as possible original electrocardiogram signals with the first duration may be segmented in each original electrocardiogram signal whose total duration is longer than or equal to the first duration. It is clear that, a number of original electrocardiogram signals with the first duration that are segmented in each original electrocardiogram signal whose total duration is longer than or equal to the first duration may alternatively be limited.

It may be understood based on the segmentation described above that, in the plurality of original electrocardiogram signals whose total duration is longer than or equal to the first duration, some original electrocardiogram signals may be segmented to obtain one original electrocardiogram signal with the first duration, and some original electrocardiogram signals may be segmented to obtain a plurality of original electrocardiogram signals with the first duration.

Regardless of the segmentation manner, the plurality of original electrocardiogram signals with the first duration are finally obtained. It should be noted that, segmentation processes of the plurality of original electrocardiogram signals with the first duration obtained by segmenting the same original electrocardiogram signal do no overlap in time domain.

In this embodiment of this application, the first duration may be $t$. It is assumed that a sampling rate of the original electrocardiogram signal is $F_s$. Then, each original electrocardiogram signal with the first duration includes $t \times F_s$ discrete data points.

Step A3: Perform low-pass filtering processing and high-pass filtering processing on original electrocardiogram signals with the first duration to obtain the plurality of electrocardiogram signal samples.

In this embodiment of this application, the low-pass filtering processing and the high-pass filtering processing may be performed on the original electrocardiogram signal with the first duration to eliminate glitches in the original electrocardiogram signal with the first duration and improve accuracy of determining a peak and a peak number subsequently.

In an actual application, the low-pass filtering processing may be performed followed by the high-pass filtering processing, or the high-pass filtering processing may be performed followed by the low-pass filtering processing.

In the low-pass filtering processing, an $\alpha$-order low-pass filter is used, where an upper frequency limit of the low-pass filter is $F_h$.

In the high-pass filtering processing, a $\beta$-order high-pass filter is used, where a lower frequency limit of the high-pass filter is $F_l$.

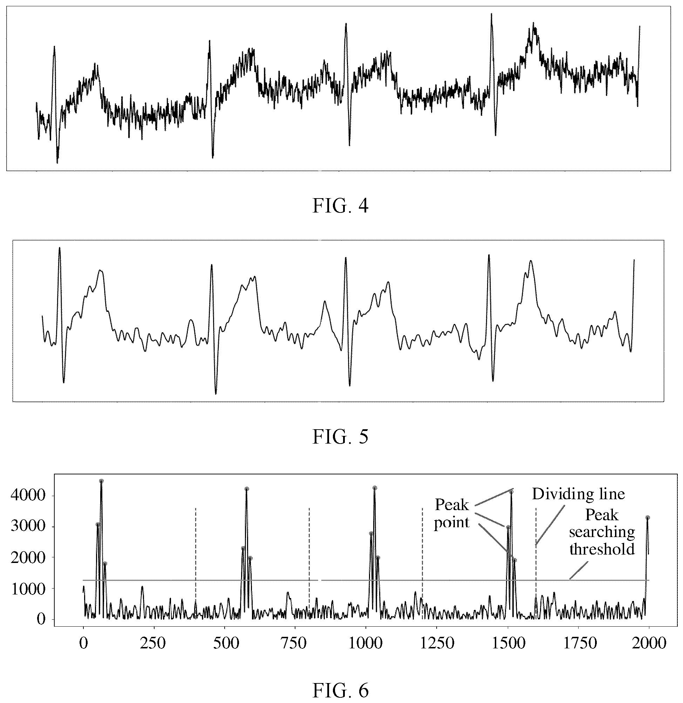

FIG. 4 shows an original electrocardiogram signal with first duration without filtering processing according to an embodiment of this application; and FIG. 5 shows an electrocardiogram signal on which filtering processing is performed according to an embodiment of this application.

It may be understood from FIG. 4 and FIG. 5 that, after filtering processing, glitches in the electrocardiogram signal are eliminated. Because features used in embodiments of this application to distinguish between different types of electrocardiogram signals are the peak and the peak number, glitches in the original electrocardiogram signal that easily causes interference on the peak and the peak number need to be removed.

In this embodiment of this application, for example, the high-pass filtering and the low-pass filtering are used to remove glitches from the original electrocardiogram signal. In an actual application, another filtering manner may also be used to eliminate glitches in the original electrocardiogram signal. This is not limited in embodiments of this application.

It should be noted that, in an actual application, signal segmentation may be first performed on the original electrocardiogram signal that is longer than or equal to the first duration, to obtain a plurality of original electrocardiogram signals with the first duration, and then filtering processing is performed on each original electrocardiogram signal with the first duration. Alternatively, filtering processing may be first performed on the original electrocardiogram signal that is longer than or equal to the first duration, to obtain each filtered original electrocardiogram signal that is longer than or equal to the first duration, and then signal segmentation is performed on each original electrocardiogram signal that is longer than or equal to the first duration and on which filtering processing is performed.

The sequence of the foregoing signal segmentation and filtering processing is not limited in embodiments of this application.

For ease of distinguishing, segmentation and filtering processing are performed on the original electrocardiogram signal to obtain the electrocardiogram signal samples. Duration of each of the electrocardiogram signal samples is the first duration, and the high-pass filtering processing and the low-pass filtering processing are performed on the electrocardiogram signal samples.

In this embodiment of this application, a number of the obtained electrocardiogram signal samples may be denoted as S.

S102: Calculate the peak variability feature and the peak number variability feature of each electrocardiogram signal sample.

This step corresponds to step A4.

In this embodiment of this application, calculation of a peak variability feature and a peak number variability feature of one of the electrocardiogram signal samples is used as an example. In an actual application, the peak variability feature and the peak number variability feature are calculated in the same manner for the electrocardiogram signal samples.

Step C1: Perform segmentation processing on an electrocardiogram signal sample to obtain a plurality of sample segments of the electrocardiogram signal sample.

The segmentation processing in this step is also a non-overlapping segmentation in time domain.

It may be set that each electrocardiogram signal sample is equally divided into $n$ sample segments not overlapping in time domain, and a number of discrete data points included in each sample segment is $\dfrac{t \times F_s}{n}$. In this embodiment of this application, $\dfrac{t \times F_s}{n}$ may be denoted as $k$. That is, each sample segment includes $k$ discrete data points.

It is assumes that an electrocardiogram signal sample may be denoted as X .After the electrocardiogram signal sample $X$ is segmented, $X = [X_1|X_2|\cdots|X_n]$, where $X_i = \left[ x_i^1, x_i^2, \cdots x_i^k \right]$, $1 \le i \le n$, $x_i^j$ represents a $j$th discrete data point in an $i$th sample segment in the electrocardiogram signal sample $X$, and $1 \le j \le k$.

Step C2: Calculate a peak list and a peak number of each sample segment.

For an $i$th sample segment $X_i$, the peak searching operation is performed in the following to obtain the peak list and the peak number of each sample segment.

First, an absolute value of a difference between each two adjacent discrete data points in the sample segment $X_i$ is calculated to obtain the absolute value of the difference $M_i = \left[ m_i^1, m_i^2, \cdots, m_i^{k-1} \right]$, where $m_i^1 = \left| x_i^2 - x_i^1 \right|$, $m_i^2 = \left| x_i^3 - x_i^2 \right|$, and $m_i^{k-1} = \left| x_i^k - x_i^{k-1} \right|$, and others are not shown in the examples.

Then, an absolute value of a difference that is greater than an absolute value of a previous difference, greater than an absolute value of a next difference, and greater than a peak searching threshold is found, and the absolute value of the difference that meets the foregoing condition is found to form a peak list.

In an example, if $m_i^\gamma > m_i^{\gamma-1}$, $m_i^\gamma > m_i^{\gamma+1}$, and $m_i^\gamma > T_M$, $m_i^\gamma$ is a peak point, where $1 < \gamma < k\text{-}1$.

A plurality of found peak points form a peak list $P_i$ of $X_i$. A number of peak points in the peak list $X_i$ is used as a peak number $S_i$ of $X_i$. $P_i = \left[ p_i^1, p_i^2, \cdots, p_i^{s_i} \right]$, where $S_i$ is used as an example of the peak number.

It is clear that, in an actual application, the foregoing peak searching operation needs to be performed on a plurality of sample segments ( $X_1$ to $X_n$ ) of the electrocardiogram signal sample $X$ to obtain the peak list and the peak number of each sample segment.

Based on the foregoing understanding, the peak list of the electrocardiogram signal sample $X$ is

$$P = \left[ P_1, P_2, \cdots, P_n \right] = \left[ p_1^1, p_1^2, \cdots, p_1^{s_1}, p_2^1, p_2^2, \cdots, p_2^{s_2}, \cdots, p_n^1, p_n^2, \cdots, p_n^{s_n} \right]$$ , where $P_i$ includes $S_i$ peak points, and the peak number of the electrocardiogram signal sample $X$ is $S = [S_1, S_2, \cdots, S_n]$, where $S_i$ is the number of peak points in the peak list represented by $P_i$.

FIG. 6 is a schematic diagram of peaks of five sample segments obtained by equally dividing the electrocardiogram signal sample $X$ into five sample segments. In FIG. 6, a dividing line is a dividing moment when the electrocardiogram signal sample $X$ is equally divided into the five sample segments; and the peak point is a peak point obtained through the peak searching operation. For a calculation process of the peak searching threshold, refer to the following descriptions.

As described above, during the peak searching operation, $m_i^\gamma$ further needs to be compared with the peak searching threshold $T_M$. Because peak searching thresholds of all sample segments of an electrocardiogram signal sample are equal when the peak searching operation is performed, the peak searching threshold is related to the electrocardiogram signal sample $X$.

In an example, the peak searching threshold is determined based on absolute values of differences respectively corresponding to a plurality of sample segments of the electrocardiogram signal sample. To be specific, the peak searching threshold is related to $M_1$ to $M_n$ of the plurality of sample segments $X_1$ to $X_n$ in the electrocardiogram signal sample $X$.

A sequence is first constructed as

$$M_{BH} = \left[ M_1, M_2, \cdots, M_n \right] = \left[ m_1^1, m_1^2, \cdots, m_1^{k-1}, m_2^1, m_2^2, \cdots, m_2^{k-1}, \cdots, m_n^1, m_n^2, \cdots, m_n^{k-1} \right]$$ , and the sequence represents a set of absolute values of differences between a plurality of sample segments of an electrocardiogram signal sample.

A first product of a first constant and a 75th percentile in the set of the absolute values of the differences of the plurality of sample segments of the electrocardiogram signal sample is calculated; a second product of a second constant and a 25th percentile in the set of the absolute values of the differences to the plurality of sample segments of the electrocardiogram signal sample; and a difference between the first product and the second product is calculated to obtain the peak searching threshold of the electrocardiogram signal sample $X$.

In an example, $T_M = a_1 \text{ * } percentile(M_{BH}, 75) - a_2 \text{ * } percentile(M_{BH}, 25)$.

$percentile(M_{BH}, 75)$ represents a 75th percentile of a sequence $M_{BH}$, to be specific, elements in the sequence

$M_{BH}$ are arranged in ascending order, then a total number $Q$ of the elements in the sequence $M_{BH}$ is calculated, and a(75% $Q$)$^{th}$ (75% times $Q$) element is selected from the sorted sequence $M_{BH}$, where *percentile*($M_{BH}$,75) is the selected element.

*percentile*($M_{BH}$, 25) may be obtained in the same way, to be specific, the elements in the sequence $M_{BH}$ are arranged in ascending order, then the total number $Q$ of the elements in the sequence $M_{BH}$ is calculated, and a(25% $Q$)$^{th}$ (25% times $Q$) element is selected from the sorted sequence $M_{BH}$, where *percentile*($M_{BH}$ ,25) is the selected element.

It is clear that, 75 and 25 in the foregoing example are only used as examples. In an actual application, the numbers may be the first percentile and the first product of the first constant, and the second percentile and the second product of the second constant. It is clear that, a more appropriate value may be selected based on a condition of the electrocardiogram signal collected by the electronic device that actually monitors the electrocardiogram signal. The value may be obtained through a plurality of experiments. Similarly, different constants may alternatively be selected as the first constant $a_1$ and the second constant $a_2$ in the foregoing example based on the situation. For example, $a_1$ may be 4, and $a_2$ may be 3.

Step C3: Obtain the peak variability feature of the electrocardiogram signal sample based on a peak list of the plurality of sample segments.

According to the foregoing example, the peak list

$$P = \left[ P_1, P_2, \cdots, P_n \right] = \left[ p_1^1, p_1^2, \cdots, p_1^{s_1}, p_2^1, p_2^2, \cdots, p_2^{s_2}, \cdots, p_n^1, p_n^2, \cdots, p_n^{s_n} \right]$$ of the electrocardiogram signal sample $X$ may be obtained.

A data feature value of the peak list of the electrocardiogram signal sample $X$ is used as the peak variability feature of the electrocardiogram signal sample.

According to the invention, a standard deviation and a mean value of peaks of the plurality of sample segments of the electrocardiogram signal sample are calculated; and

a ratio of the standard deviation to the mean value of the peaks of the plurality of sample segments are calculated to obtain the peak variability feature of the electrocardiogram signal sample.

That is,

$$f_1 = \frac{std(P)}{mean(P)}.$$

$f_1$ represents the peak variability feature of the electrocardiogram signal sample $X$, $std(P)$ represents a standard deviation of peaks in the peak list $P$, and $mean(P)$ represents a mean value of the peaks in the peak list $P$.

Step C4: Obtain the peak number variability feature of the electrocardiogram signal sample based on the peak numbers of the plurality of sample segments.

According to the foregoing example, peak numbers $S = [S_1, S_2, \cdots, S_n]$ of the electrocardiogram signal sample $X$ may be obtained.

A data feature value of the peak numbers of the electrocardiogram signal sample $X$ is used as the peak number variability feature of the electrocardiogram signal sample.

According to the invention, a difference between the largest value and the smallest value of the peak numbers of the plurality of sample segments of the electrocardiogram signal sample is calculated;

a median value of the peak numbers of the plurality of sample segments of the electrocardiogram signal sample is calculated; and

a ratio of the difference to the median value is calculated to obtain the peak number variability feature of the electrocardiogram signal sample.

That is,

$$f_2 = \frac{\max(S) - \min(S)}{median(S)}.$$

$f_2$ represents the peak number variability feature of the electrocardiogram signal sample $X$, max($S$) represents the largest value of the peak numbers in a peak number set $S$, min($S$) represents the smallest value of the peak numbers in the peak number set $S$, and $median(S)$ represents a median value of the peak numbers in the peak number set $S$.

Through the foregoing manner, the peak variability feature and the peak number variability feature of the electrocardiogram signal sample with the first duration may be obtained.

S103: Obtain the first threshold based on peak variability features of the plurality of electrocardiogram signal samples; and obtain the second threshold based on peak number variability features of the plurality of electrocardiogram signal samples.

This step corresponds to step A5 in the embodiment shown in FIG. 2.

It is assumed that $W$ electrocardiogram signal samples are used as learning samples in this embodiment of this application. Then, $W$ peak variability features and $W$ peak number variability features may be obtained based on the W electrocardiogram signal samples in this embodiment of this application.

Correspondingly, a peak variability feature set $F_1 = \left[ f_1^1, f_1^2, \cdots, f_1^W \right]$ ; and a peak number variability set $F_2 = \left[ f_2^1, f_2^2, \cdots, f_2^W \right]$ .

Then, a data feature value of the peak variability feature set is calculated to obtain the abnormal dividing point (the first threshold) of the peak variability features.

In an example, a third product of a third percentile and a third constant in the peak variability features of the plurality of electrocardiogram signal samples is calculated;

a fourth product of a fourth constant and a fourth percentile in the peak variability features of the plurality of electrocardiogram signal samples is calculated; and

a difference between the third product and the fourth product is calculated to obtain the first threshold.

This embodiment of this application is described by using an example in which the third percentile is the 75th percentile, the fourth percentile is the 25th percentile, the third constant is 4, and the fourth constant is 3.

$$U_1 = 4 \times percentile(F_1, 75) - 3 \times percentile(F_1, 25) .$$

$U_1$ is the first threshold representing the abnormal dividing point of the peak variability features.

In the same way, the abnormal dividing point (the second threshold) of the peak number variability features may be obtained.

In an example, a fifth product of a fifth constant and a fifth percentile in the peak number variability features of the plurality of electrocardiogram signal samples is calculated;

a sixth product of a sixth constant and a sixth percentile in the peak number variability features of the plurality of electrocardiogram signal samples is calculated; and

a difference between the fifth product and the sixth product is calculated to obtain the second threshold.

This embodiment of this application is described by using an example in which the fifth percentile is the 75th percentile, the sixth percentile is the 25th percentile, the fifth constant is 4, and the sixth constant is 3.

$$U_2 = 4 \times percentile(F_2, 75) - 3 \times percentile(F_2, 25) .$$

$U_2$ is the first threshold representing the abnormal dividing point of the peak number variability features.

In this way, the first threshold and the second threshold for classifying types of monitored electrocardiogram signals can be determined.

After the first threshold and the second threshold are determined, the first threshold, the second threshold, and an algorithm model for calculating the peak variability feature and the peak number variability feature are preset in the electronic device. The electronic device can implement real-time detection of the electrocardiogram signal, as well as quality evaluation of the electrocardiogram signal.

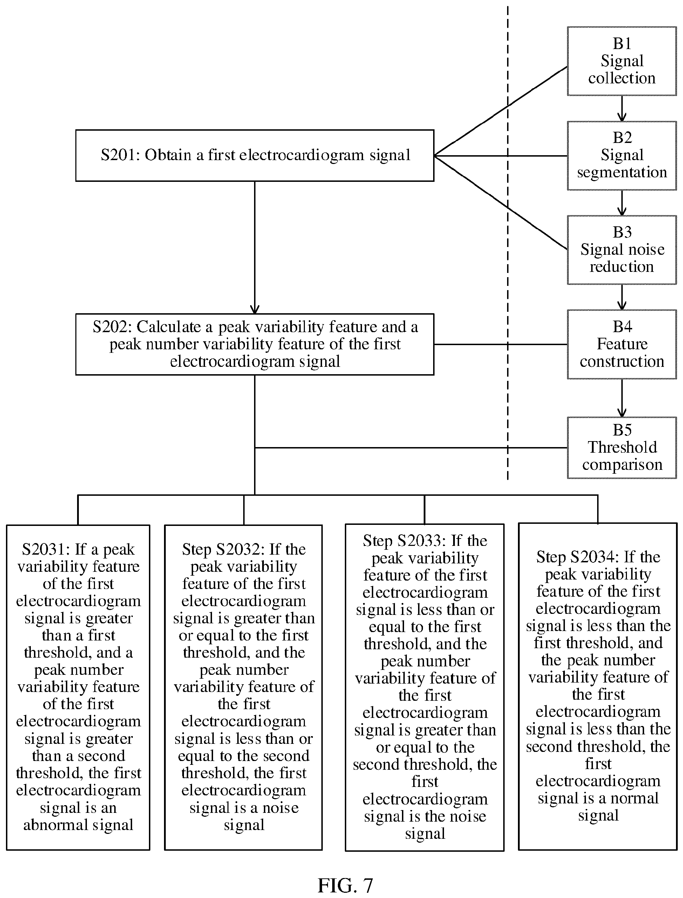

FIG. 7 is a schematic flowchart of an electrocardiogram signal quality evaluation method according to an embodiment of this application. This schematic flowchart is based on the flowchart shown in FIG. 3 and corresponds to step B1 to step B5 in the embodiment shown in FIG. 2.

Step S201: Obtain a first electrocardiogram signal.

In this embodiment of this application, the electrocardiogram signal may be measured by using an electrocardiogram monitoring apparatus on an electronic device, and then the electronic device processes the electrocardiogram signal to evaluate a quality of the electrocardiogram signal.

In an actual application, it is also possible that, after the electrocardiogram signal is measured by the electrocardiogram monitoring apparatus, the electrocardiogram monitoring apparatus sends the measured electrocardiogram signal to an electronic device for performing electrocardiogram signal quality evaluation, and another electronic device performs the quality evaluation, that is, the electrocardiogram signal monitoring and an electrocardiogram signal quality evaluation process may be respectively set up on different electronic devices. This is not limited in embodiments of this application.

In another embodiment of this application, the obtaining a first electrocardiogram signal includes:

obtaining an original electrocardiogram signal with first duration; and

performing low-pass filtering processing and high-pass filtering processing on the original electrocardiogram signal to obtain the first electrocardiogram signal.

In this embodiment of this application, after the electrocardiogram signal is measured by the electrocardiogram monitoring apparatus, the electrocardiogram signal is the original electrocardiogram signal. The electronic device may perform segmentation processing and filtering processing on the measured original electrocardiogram signal to obtain the first electrocardiogram signal. This step corresponds to step B1 to step B3.

The first electrocardiogram signal is a filtered electrocardiogram signal with first duration. The duration of the electrocardiogram signal in the test stage is consistent with that of the electrocardiogram signal in a learning stage, thereby ensuring that the learned first threshold and second threshold may be used to perform quality evaluation on the electrocardiogram signal.

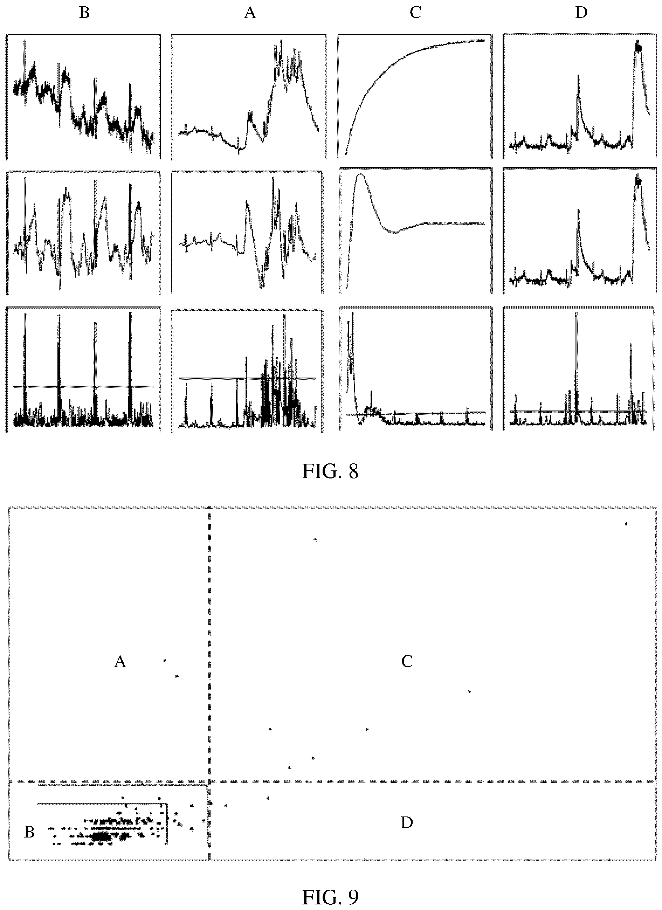

FIG. 8 is a schematic diagram of waveform changes in a processing process of four electrocardiogram signals (A, B, C, and D) according to an embodiment of this application. Each electrocardiogram signal separately shows that: the original electrocardiogram signal with the first duration (in the first line of FIG. 8), the first electrocardiogram signal obtained by filtering processing (in the second line of FIG. 8), and a peak point after peak searching operation (in the third line of FIG. 8).

Step S202: Calculate a peak variability feature and a peak number variability feature of the first electrocardiogram signal.

In this embodiment of this application, this step corresponds to step B4 in the embodiment shown in FIG. 2.

A manner of calculating the peak variability feature of the first electrocardiogram signal is the same as a manner of calculating the peak variability feature of an electrocardiogram signal sample with first duration.

Similarly, a manner of calculating the peak number variability feature of the first electrocardiogram signal is the same as a manner of calculating the peak number variability feature of an electrocardiogram signal sample with first duration.

In another embodiment of this application, the calculating a peak variability feature and a peak number variability feature of the first electrocardiogram signal includes:

performing segmentation processing on the first electrocardiogram signal to obtain a plurality of signal segments;

calculating a peak list and a peak number of each signal segment, where the peak list includes N peaks, and N represents the peak number;

obtaining the peak variability feature of the first electrocardiogram signal based on peak lists of the plurality of signal segments; and

obtaining the peak number variability feature of the first electrocardiogram signal based on peak numbers of the plurality of signal segments.

In this embodiment of this application, the signal segment includes a plurality of discrete data points, and the signal segment is equivalent to a sample segment in a learning stage. For details, reference may be made to the description of the sample segment in the foregoing embodiments, and details are not described herein again.

In another embodiment of this application, the calculating a peak list of each signal segment includes: calculating an absolute value of a difference between each two adjacent data points in a first signal segment, where the first signal segment is any signal segment in the signal segments, a peak list of the first signal segment includes one or more first peaks, the first peak is an absolute value of a difference that is greater than an absolute value of a previous difference, greater than an absolute value of a next difference, and greater than a peak searching threshold, and the peak searching threshold is determined based on absolute values of differences respectively corresponding to the plurality of signal segments of the first electrocardiogram signal.

In another embodiment of this application, the method for determining the peak searching threshold includes:

calculating a first product of a first constant and a first percentile in a set of the absolute values of the differences corresponding to the plurality of signal segments of the first electrocardiogram signal;

calculating a second product of a second constant and a second percentile in the set of the absolute values of the differences corresponding to the plurality of signal segments of the first electrocardiogram signal; and

calculating a difference between the first product and the second product to obtain the peak searching threshold.

In another embodiment of this application, the obtaining the peak variability feature of the first electrocardiogram signal based on peak lists of the plurality of signal segments includes:

calculating a standard deviation and a mean value of peaks of the plurality of signal segments of the first electrocardiogram signal; and
calculating a ratio of the standard deviation to the mean value of the peaks of the plurality of signal segments to obtain the peak variability feature of the first electrocardiogram signal.

In another embodiment of this application, the obtaining the peak number variability feature of the first electrocardiogram signal based on peak numbers of the plurality of signal segments includes:

calculating a first difference between a largest value and a smallest value of the peak numbers of the plurality of signal segments of the first electrocardiogram signal;
calculating a median value of the peak numbers of the plurality of signal segments of the first electrocardiogram signal; and
calculating a ratio of the first difference value to the median value to obtain the peak number variability feature of the first electrocardiogram signal.

For a specific implementation process of calculating the peak variability feature and the peak number variability feature of the first electrocardiogram signal, reference may be made to a specific implementation process of calculating the peak variability feature and the peak number variability feature of an electrocardiogram signal sample in the foregoing embodiments, and details are not described herein again.

Step S2031: If a peak variability feature of the first electrocardiogram signal is greater than a first threshold, and a peak number variability feature of the first electrocardiogram signal is greater than a second threshold, the first electrocardiogram signal is an abnormal signal.

Step S2032: If the peak variability feature of the first electrocardiogram signal is greater than or equal to the first threshold, and the peak number variability feature of the first electrocardiogram signal is less than or equal to the second threshold, the first electrocardiogram signal is a noise signal.

Step S2033: If the peak variability feature of the first electrocardiogram signal is less than or equal to the first threshold, and the peak number variability feature of the first electrocardiogram signal is greater than or equal to the second threshold, the first electrocardiogram signal is the noise signal.

Step S2034: If the peak variability feature of the first electrocardiogram signal is less than the first threshold, and the peak number variability feature of the first electrocardiogram signal is less than the second threshold, the first electrocardiogram signal is a normal signal.

FIG. 9 is an evaluation result obtained by performing quality evaluation on electrocardiogram signals A, B, C, and D after a threshold comparison at step B5.

A two-dimensional feature plane is divided into 4 regions through the first threshold and the second threshold, namely, a normal region, an abnormal region, and a noise region.

If the peak variability feature of the electrocardiogram signal B is less than the first threshold, and the peak number variability feature of the electrocardiogram signal B is less than the second threshold, the electrocardiogram signal B is the normal signal.

If the peak variability feature of the electrocardiogram signal C is greater than the first threshold, and the peak number variability feature of the electrocardiogram signal C is greater than the second threshold, the electrocardiogram signal C is the abnormal signal.

If the peak variability feature of the electrocardiogram signal A is greater than or equal to the first threshold, and the peak number variability feature of the electrocardiogram signal A is less than or equal to the second threshold, the electrocardiogram signal A is the noise signal.

If the peak variability feature of the electrocardiogram signal D is less than or equal to the first threshold, and the peak number variability feature of the electrocardiogram signal D is greater than or equal to the second threshold, the electrocardiogram signal D is the noise signal.

It is clear that, in an actual application, a plurality of signal segments may be obtained during or after acquisition of the electrocardiogram signal. After the plurality of signal segments are evaluated, a signal segment located in a region B is the normal signal, a signal segment located in a region C is the abnormal signal, and signal segments located in a region A and a region D are noise segments. Through this method, the electrocardiogram signal classification may be accurately evaluated.

It should be understood that the order of the sequence numbers of the steps in the foregoing embodiments does not mean the order of execution, and the execution order of each process is determined based on functions and internal logic of the process, and should not be construed as any limitation on the implementation process of embodiments of this application.

An embodiment of this application further provides a computer-readable storage medium, where the computer-readable storage medium stores a computer program, and the computer program, when executed by a processor, implements the steps in the foregoing method embodiments.

An embodiment of this application further provides a computer program product, the computer program product, when run on an electronic device, causes the electronic device to implement the steps in the foregoing method embodiments.

When an integrated unit is implemented in the form of a software functional unit and sold or used as an independent product, the integrated unit may be stored in a computer-readable storage medium. Based on such understanding, all or some of the processes of the methods embodiments of this application may be implemented by a computer program indicating relevant hardware. The computer program may be stored in a computer-readable storage medium. During execution of the computer program by the processor, steps of the foregoing method embodiments may be implemented. The computer program includes computer program code. The computer program code may be in source code form, object code form, executable file or some intermediate forms, or the like. The computer-readable medium may include at least: any entity or apparatus that is capable of carrying the computer program code to a first device, a recording medium, a computer memory, a read-only memory (Read-Only Memory, ROM), a random access memory (Random PROM Memory, RAM), an electric carrier signal, a telecommunication signal, or a software distribution medium. For example, a USB flash drive, a removable hard disk, a magnetic disk, or a compact disc. In some jurisdictions, according to legislation and patent practice, the computer-readable medium may not be an electric carrier signal and a telecommunication signal.

An embodiment of this application further provides a chip system, where the chip system includes a processor. The processor is coupled to a memory and executes a computer program stored in the memory, to implement the steps according to any method embodiment of this application. The chip system may be a single chip or a chip module including a plurality of chips.

In the foregoing embodiment, descriptions of the embodiments have different emphases. As for parts that are not described in detail in one embodiment, reference may be made to the relevant descriptions of other embodiments.

A person of ordinary skill in the art may be aware that the exemplary units and method steps described with reference to the embodiments disclosed in this specification can be implemented in electronic hardware, or a combination of computer software and electronic hardware. Whether the functions are performed by hardware or software depends on particular applications and design constraints of the technical solutions. A person skilled in the art may use different methods to implement the described functions for each particular application, but it should not be considered that the implementation goes beyond the scope of this application.

The foregoing embodiments are merely used for describing the technical solutions of this application, but are not intended to limit this application. Although this application is described in detail with reference to the foregoing embodiments, it should be appreciated by a person skilled in the art that, modifications may still be made to the technical solutions described in the foregoing embodiments, or equivalent replacements may be made to the part of the technical features; and as long as such modifications or replacements do not cause the essence of corresponding technical solutions to depart from the scope of the technical solutions defined in the claims.

**Claims**

1. A computer implemented electrocardiogram signal quality evaluation method using an electronic device (100) for evaluating whether an electrocardiogram signal is a normal signal, comprising:

   obtaining (S201), by a functional module in the electronic device (100), a first electrocardiogram signal; and
   calculating (S202), by the functional module in the electronic device (100), a peak variability feature and a peak number variability feature of the first electrocardiogram signal,
   wherein the peak variability feature is obtained by calculating a ratio between a standard deviation and a mean value of peaks in a plurality of sample segments of the first electrocardiogram signal obtained by performing a signal segmentation in time domain using a pre-set duration on the first electrocardiogram signal;
   wherein the peak number variability feature is obtained by calculating a ratio of the difference between the largest value of the peak numbers in a sample segment of the plurality of sample segments and the smallest value of the peak numbers in a sample segment of the plurality of sample segments to a median value of the peak numbers in a sample segment of the plurality of sample segments;
   wherein if the peak variability feature of the first electrocardiogram signal is less than a first threshold, and the peak number variability feature of the first electrocardiogram signal is less than a second threshold, determining (S2034), by the functional module in the electronic device (100), that the first electrocardiogram signal is a normal signal;

wherein the first threshold represents an abnormal dividing point of the peak variability feature, the first threshold is determined based on peak variability features of a plurality of electrocardiogram signal samples, wherein the second threshold represents an abnormal dividing point of the peak number variability feature, and the second threshold is determined based on peak number variability features of the plurality of electrocardiogram signal samples.

2. The method according to claim 1, wherein the obtaining a first electrocardiogram signal comprises:

    obtaining an original electrocardiogram signal with first duration; and
    performing filtering processing on the original electrocardiogram signal to obtain the first electrocardiogram signal.

3. The method according to claim 2, wherein the performing filtering processing on the original electrocardiogram signal comprises:
performing low-pass filtering processing and high-pass filtering processing on the original electrocardiogram signal.

4. The method according to any one of claims 1 to 3, wherein the calculating a peak variability feature and a peak number variability feature of the first electrocardiogram signal comprises:

    performing segmentation processing on the first electrocardiogram signal to obtain the plurality of signal segments;
    calculating a peak list and a peak number of each signal segment, wherein the peak list comprises N peaks, and N represents the peak number;
    obtaining the peak variability feature of the first electrocardiogram signal based on peak lists of the plurality of signal segments; and
    obtaining the peak number variability feature of the first electrocardiogram signal based on peak numbers of the plurality of signal segments.

5. The method according to claim 4, wherein the signal segment comprises a plurality of discrete data points, and the calculating a peak list of each signal segment comprises:
calculating an absolute value of a difference between each two adjacent data points in a first signal segment, wherein the first signal segment is any signal segment in the signal segments, a peak list of the first signal segment comprises one or more first peaks, the first peak is an absolute value of a difference that is greater than an absolute value of a previous difference, greater than an absolute value of a next difference, and greater than the peak searching threshold, and the peak searching threshold is determined based on absolute values of differences respectively corresponding to the plurality of signal segments of the first electrocardiogram signal.

6. The method according to claim 5, wherein a manner for determining the peak searching threshold comprises:

    calculating a first product of a first constant and a first percentile in a set of the absolute values of the differences corresponding to the plurality of signal segments of the first electrocardiogram signal;
    calculating a second product of a second constant and a second percentile in the set of the absolute values of the differences corresponding to the plurality of signal segments of the first electrocardiogram signal; and
    calculating a difference between the first product and the second product to obtain the peak searching threshold.

7. The method according to any one of claims 4 to 6, wherein the obtaining the peak variability feature of the first electrocardiogram signal based on peak lists of the plurality of signal segments comprises:

    calculating the standard deviation and the mean value of peaks of the plurality of signal segments of the first electrocardiogram signal; and
    calculating the ratio of the standard deviation to the mean value of the peaks of the plurality of signal segments to obtain the peak variability feature of the first electrocardiogram signal.

8. The method according to any one of claims 4 to 6, wherein the obtaining the peak number variability feature of the first electrocardiogram signal based on peak numbers of the plurality of signal segments comprises:

    calculating a first difference between the largest value and the smallest value of the peak numbers of the plurality of signal segments of the first electrocardiogram signal;

calculating the median value of the peak numbers of the plurality of signal segments of the first electrocardiogram signal; and

calculating the ratio of the first difference value to the median value to obtain the peak number variability feature of the first electrocardiogram signal.

9. The method according to any one of claims 1 to 8, wherein a manner of determining the first threshold and the second threshold comprises:

obtaining the plurality of electrocardiogram signal samples, wherein duration of each of the plurality of electrocardiogram signal samples is the first duration;
calculating a peak variability feature and a peak number variability feature of each electrocardiogram signal sample;
obtaining the first threshold based on the peak variability features of the plurality of electrocardiogram signal samples; and
obtaining the second threshold based on the peak number variability features of the plurality of electrocardiogram signal samples.

10. The method according to claim 9, wherein the obtaining the first threshold based on the peak variability features of the plurality of electrocardiogram signal samples comprises:

calculating a third product of a third constant and a third percentile in the peak variability features of the plurality of electrocardiogram signal samples;
calculating a fourth product of a fourth constant and a fourth percentile in the peak variability features of the plurality of electrocardiogram signal samples; and
calculating a difference between the third product and the fourth product to obtain the first threshold.

11. The method according to claim 9, wherein the obtaining the second threshold based on the peak number variability features of the plurality of electrocardiogram signal samples comprises:

calculating a fifth product of a fifth constant and a fifth percentile in the peak number variability features of the plurality of electrocardiogram signal samples;
calculating a sixth product of a sixth constant and a sixth percentile in the peak number variability features of the plurality of electrocardiogram signal samples; and
calculating a difference between the fifth product and the sixth product to obtain the second threshold.

12. The method according to any one of claims 1 to 11, further comprising:

if the peak variability feature of the first electrocardiogram signal is greater than the first threshold, and the peak number variability feature of the first electrocardiogram signal is greater than the second threshold, determining (S2031) that the first electrocardiogram signal is an abnormal signal;
if the peak variability feature of the first electrocardiogram signal is greater than or equal to the first threshold, and the peak number variability feature of the first electrocardiogram signal is less than or equal to the second threshold, determining (S2032) that the first electrocardiogram signal is a noise signal; or
if the peak variability feature of the first electrocardiogram signal is less than or equal to the first threshold, and the peak number variability feature of the first electrocardiogram signal is greater than or equal to the second threshold, determining (S2033) that the first electrocardiogram signal is a noise signal.

13. The method according to any one of claims 9 to 12, wherein the obtaining the plurality of electrocardiogram signal samples comprises:

obtaining a plurality of original electrocardiogram signals;
performing data segmentation on the plurality of original electrocardiogram signals to obtain a plurality of original electrocardiogram signals with the first duration; and
performing low-pass filtering processing and high-pass filtering processing on the original electrocardiogram signals with the first duration to obtain the plurality of electrocardiogram signal samples.

14. The method according to any one of claims 9 to 12, wherein a manner of calculating the peak variability feature of each electrocardiogram signal sample is the same as a manner of calculating the peak variability feature of the first electrocardiogram signal; and

a manner of calculating the peak number variability feature of each electrocardiogram signal sample is the same as a manner of calculating the peak number variability feature of the first electrocardiogram signal.

15. An electronic device (100), comprising a processor (110), wherein the processor is configured to run a computer program stored in a memory (121), to cause the electronic device to implement the method according to any one of claims 1 to 14.

**Patentansprüche**

1. Ein computerimplementiertes Verfahren zur Bewertung der Signalqualität eines Elektrokardiogramms unter Verwendung eines elektronischen Geräts (100) zur Bewertung, ob ein Elektrokardiogrammsignal ein normales Signal ist, umfassend:

Erhalten (S201) eines ersten Elektrokardiogrammsignals durch ein Funktionsmodul im elektronischen Gerät (100); und

Berechnen (S202) eines Spitzenvariabilitätsmerkmals und eines Spitzenanzahl-Variabilitätsmerkmals des ersten Elektrokardiogrammsignals durch das Funktionsmodul im elektronischen Gerät (100),

wobei das Spitzenvariabilitätsmerkmal durch Berechnung des Verhältnisses zwischen der Standardabweichung und dem Mittelwert der Spitzen in einer Vielzahl von Probensegmenten des ersten Elektrokardiogrammsignals erhalten wird, wobei die Probensegmente durch eine Signalsplittung im Zeitbereich mittels einer vorgegebenen Dauer am ersten Elektrokardiogrammsignal gebildet werden;

wobei das Spitzenanzahl-Variabilitätsmerkmal durch Berechnung des Verhältnisses der Differenz zwischen dem größten Wert der Spitzenzahlen in einem Probensegment der Vielzahl von Probensegmenten und dem kleinsten Wert der Spitzenzahlen in einem Probensegment der Vielzahl von Probensegmenten zum Medianwert der Spitzenzahlen in einem Probensegment der Vielzahl von Probensegmenten erhalten wird;

wobei, wenn das Spitzenvariabilitätsmerkmal des ersten Elektrokardiogrammsignals kleiner als ein erster Schwellenwert ist und das Spitzenanzahl-Variabilitätsmerkmal des ersten Elektrokardiogrammsignals kleiner als ein zweiter Schwellenwert ist, das Funktionsmodul im elektronischen Gerät (100) bestimmt (S2034), dass das erste Elektrokardiogrammsignal ein normales Signal ist;

wobei der erste Schwellenwert einen abnormalen Trennpunkt des Spitzenvariabilitätsmerkmals darstellt, und der erste Schwellenwert basierend auf den Spitzenvariabilitätsmerkmalen einer Vielzahl von Elektrokardiogrammsignalproben bestimmt wird,

wobei der zweite Schwellenwert einen abnormalen Trennpunkt des Spitzenanzahl-Variabilitätsmerkmals darstellt und der zweite Schwellenwert basierend auf den Spitzenanzahl-Variabilitätsmerkmalen der Vielzahl von Elektrokardiogrammsignalproben bestimmt wird.

2. Verfahren nach Anspruch 1, wobei das Erhalten eines ersten Elektrokardiogrammsignals umfasst:

Erhalten eines ursprünglichen Elektrokardiogrammsignals mit erster Dauer; und

Durchführen einer Filterverarbeitung am ursprünglichen Elektrokardiogrammsignal zum Erhalt des ersten Elektrokardiogrammsignals.

3. Verfahren nach Anspruch 2, wobei die Durchführung der Filterverarbeitung am ursprünglichen Elektrokardiogrammsignal umfasst:

Durchführen einer Tiefpassfilterung und einer Hochpassfilterung am ursprünglichen Elektrokardiogrammsignal.

4. Verfahren nach einem der Ansprüche 1 bis 3, wobei die Berechnung eines Spitze-Variabilitätsmerkmals und eines Spitzenanzahl-Variabilitätsmerkmals des ersten Elektrokardiogrammsignals Folgendes umfasst:

Durchführen einer Segmentierungsverarbeitung des ersten Elektrokardiogrammsignals, um die Vielzahl von Signalsegmenten zu erhalten;

Berechnen einer Spitzenliste und einer Spitzenanzahl jedes Signalsegments, wobei die Spitzenliste N Spitzen umfasst und N die Spitzenanzahl darstellt;

Ermitteln des Spitze-Variabilitätsmerkmals des ersten Elektrokardiogrammsignals basierend auf den Spitzenlisten der Vielzahl von Signalsegmenten; und

Ermitteln des Spitzenanzahl-Variabilitätsmerkmals des ersten Elektrokardiogrammsignals basierend auf den Spitzenanzahlen der Vielzahl von Signalsegmenten.

5. Verfahren nach Anspruch 4, wobei das Signalsegment eine Vielzahl von diskreten Datenpunkten umfasst, und das Berechnen einer Spitzenliste jedes Signalsegments Folgendes umfasst:
Berechnen eines Absolutwertes der Differenz zwischen jeweils zwei benachbarten Datenpunkten in einem ersten Signalsegment, wobei das erste Signalsegment ein beliebiges Signalsegment der Signalsegmente ist, eine Spitzenliste des ersten Signalsegments ein oder mehrere erste Spitzen umfasst, die erste Spitze ein Absolutwert einer Differenz ist, der größer ist als der Absolutwert der vorherigen Differenz, größer als der Absolutwert der nächsten Differenz und größer als der Spitzensuchschwellenwert, wobei der Spitzensuchschwellenwert basierend auf den Absolutwerten der Differenzen bestimmt wird, die jeweils den Vielzahl von Signalsegmenten des ersten Elektrokardiogrammsignals entsprechen.

6. Verfahren nach Anspruch 5, wobei eine Art zur Bestimmung des Spitzensuchschwellenwerts Folgendes umfasst:

Berechnen eines ersten Produkts aus einer ersten Konstante und einem ersten Perzentil in einer Menge der Absolutwerte der Differenzen, die den Vielzahl von Signalsegmenten des ersten Elektrokardiogrammsignals entsprechen;
Berechnen eines zweiten Produkts aus einer zweiten Konstante und einem zweiten Perzentil in der Menge der Absolutwerte der Differenzen, die den Vielzahl von Signalsegmenten des ersten Elektrokardiogrammsignals entsprechen; und
Berechnen einer Differenz zwischen dem ersten Produkt und dem zweiten Produkt, um den Spitzensuchschwellenwert zu erhalten.

7. Verfahren nach einem der Ansprüche 4 bis 6, wobei das Ermitteln des Spitze-Variabilitätsmerkmals des ersten Elektrokardiogrammsignals basierend auf den Spitzenlisten der Vielzahl von Signalsegmenten Folgendes umfasst:

Berechnen der Standardabweichung und des Mittelwerts der Spitzen der Vielzahl von Signalsegmenten des ersten Elektrokardiogrammsignals; und
Das Verhältnis der Standardabweichung zum Mittelwert der Spitzen der Vielzahl von Signalabschnitten berechnen, um die Spitzenvariabilitätskennzahl des ersten Elektrokardiogrammsignals zu erhalten.

8. Verfahren nach einem der Ansprüche 4 bis 6, wobei das Erlangen der Spitzenanzahlvariabilitätskennzahl des ersten Elektrokardiogrammsignals auf der Grundlage der Spitzenzahlen der Vielzahl von Signalabschnitten Folgendes umfasst:

Berechnen einer ersten Differenz zwischen dem größten Wert und dem kleinsten Wert der Spitzenzahlen der Vielzahl von Signalabschnitten des ersten Elektrokardiogrammsignals;
Berechnen des Medianwerts der Spitzenzahlen der Vielzahl von Signalabschnitten des ersten Elektrokardiogrammsignals; und
Berechnen des Verhältnisses des Wertes der ersten Differenz zum Medianwert, um die Spitzenanzahlvariabilitätskennzahl des ersten Elektrokardiogrammsignals zu erhalten.

9. Verfahren nach einem der Ansprüche 1 bis 8, wobei ein Verfahren zur Bestimmung der ersten Schwelle und der zweiten Schwelle Folgendes umfasst:

Erhalt der Vielzahl von Elektrokardiogramm-Signalproben, wobei die Dauer jeder der Vielzahl von Elektrokardiogramm-Signalproben der ersten Dauer entspricht;
Berechnen einer Spitzenvariabilitätskennzahl und einer Spitzenanzahlvariabilitätskennzahl für jede Elektrokardiogramm-Signalprobe;
Erhalt der ersten Schwelle auf Basis der Spitzenvariabilitätskennzahlen der Vielzahl von Elektrokardiogramm-Signalproben; und
Erhalt der zweiten Schwelle auf Basis der Spitzenanzahlvariabilitätskennzahlen der Vielzahl von Elektrokardiogramm-Signalproben.

10. Verfahren nach Anspruch 9, wobei das Erlangen der ersten Schwelle auf Basis der Spitzenvariabilitätskennzahlen der Vielzahl von Elektrokardiogramm-Signalproben Folgendes umfasst:

Berechnen eines dritten Produkts aus einer dritten Konstante und einem dritten Perzentil innerhalb der Spitzenvariabilitätskennzahlen der Vielzahl von Elektrokardiogramm-Signalproben;

Berechnen eines vierten Produkts aus einer vierten Konstante und einem vierten Perzentil innerhalb der Spitzenvariabilitätskennzahlen der Vielzahl von Elektrokardiogramm-Signalproben; und

Berechnen einer Differenz zwischen dem dritten Produkt und dem vierten Produkt, um die erste Schwelle zu erhalten.

11. Verfahren nach Anspruch 9, wobei das Erlangen der zweiten Schwelle auf Basis der Spitzenanzahlvariabilitätskennzahlen der Vielzahl von Elektrokardiogramm-Signalproben Folgendes umfasst:

Berechnen eines fünften Produkts aus einer fünften Konstante und einem fünften Perzentil innerhalb der Spitzenanzahlvariabilitätskennzahlen der Vielzahl von Elektrokardiogramm-Signalproben;

Berechnung eines sechsten Produkts aus einer sechsten Konstante und einem sechsten Perzentil in den Merkmalsvariationen der Spitzenzahl der Vielzahl von Elektrokardiogramm-Signalproben; und

Berechnung einer Differenz zwischen dem fünften Produkt und dem sechsten Produkt zur Ermittlung des zweiten Schwellenwerts.

12. Verfahren gemäß einem der Ansprüche 1 bis 11, wobei ferner umfasst ist:

Wenn das Spitzenvariabilitätsmerkmal des ersten Elektrokardiogrammsignals größer als der erste Schwellenwert ist und das Spitzenzahl-Variabilitätsmerkmal des ersten Elektrokardiogrammsignals größer als der zweite Schwellenwert ist, wird bestimmt (S2031), dass das erste Elektrokardiogrammsignal ein abnormales Signal ist;

Wenn das Spitzenvariabilitätsmerkmal des ersten Elektrokardiogrammsignals größer oder gleich dem ersten Schwellenwert ist und das Spitzenzahl-Variabilitätsmerkmal des ersten Elektrokardiogrammsignals kleiner oder gleich dem zweiten Schwellenwert ist, wird bestimmt (S2032), dass das erste Elektrokardiogrammsignal ein Störsignal ist; oder

Wenn das Spitzenvariabilitätsmerkmal des ersten Elektrokardiogrammsignals kleiner oder gleich dem ersten Schwellenwert ist und das Spitzenzahl-Variabilitätsmerkmal des ersten Elektrokardiogrammsignals größer oder gleich dem zweiten Schwellenwert ist, wird bestimmt (S2033), dass das erste Elektrokardiogrammsignal ein Störsignal ist.

13. Verfahren gemäß einem der Ansprüche 9 bis 12, wobei das Erhalten der Vielzahl von Elektrokardiogramm-Signalproben Folgendes umfasst:

Erhalten einer Vielzahl von ursprünglichen Elektrokardiogrammsignalen;

Durchführen einer Daten-Segmentierung der Vielzahl von ursprünglichen Elektrokardiogrammsignalen zur Erzeugung einer Vielzahl von ursprünglichen Elektrokardiogrammsignalen mit der ersten Dauer; und

Durchführen einer Tiefpass- und Hochpassfilterverarbeitung der ursprünglichen Elektrokardiogrammsignale mit der ersten Dauer, um die Vielzahl von Elektrokardiogramm-Signalproben zu erhalten.

14. Verfahren gemäß einem der Ansprüche 9 bis 12, wobei das Verfahren zur Berechnung des Spitzenvariabilitätsmerkmals jeder Elektrokardiogrammsignalprobe dasselbe ist wie das Verfahren zur Berechnung des Spitzenvariabilitätsmerkmals des ersten Elektrokardiogrammsignals; und

Das Verfahren zur Berechnung des Spitzenzahl-Variabilitätsmerkmals jeder Elektrokardiogrammsignalprobe entspricht dem Verfahren zur Berechnung des Spitzenzahl-Variabilitätsmerkmals des ersten Elektrokardiogrammsignals.

15. Elektronisches Gerät (100), einschließlich eines Prozessors (110), wobei der Prozessor so konfiguriert ist, dass er ein in einem Speicher (121) gespeichertes Computerprogramm ausführt, um das elektronische Gerät dazu zu veranlassen, das Verfahren gemäß einem der Ansprüche 1 bis 14 zu implementieren.

## Revendications

1. Procédé d'évaluation de la qualité de signal électrocardiographique mis en œuvre par ordinateur utilisant un dispositif électronique (100) pour évaluer si un signal électrocardiographique est un signal normal, comprenant :

l'obtention (S201), par un module fonctionnel dans le dispositif électronique (100), d'un premier signal électrocardiographique ; et

le calcul (S202), par le module fonctionnel dans le dispositif électronique (100), d'une caractéristique de variabilité des pics et d'une caractéristique de variabilité du nombre de pics du premier signal électrocardiographique,

la caractéristique de variabilité des pics étant obtenue en calculant le rapport entre l'écart type et la valeur moyenne des pics dans une pluralité de segments d'échantillons du premier signal électrocardiographique obtenus par une segmentation du signal dans le domaine temporel à l'aide d'une durée prédéfinie sur le premier signal électrocardiographique ;

la caractéristique de variabilité du nombre de pics étant obtenue en calculant le rapport entre la différence entre la valeur maximale du nombre de pics dans un segment d'échantillon de la pluralité de segments d'échantillons et la valeur minimale du nombre de pics dans un segment d'échantillon de la pluralité de segments d'échantillons, et la valeur médiane du nombre de pics dans un segment d'échantillon de la pluralité de segments d'échantillons ;

lorsque la caractéristique de variabilité des pics du premier signal électrocardiographique est inférieure à un premier seuil, et la caractéristique de variabilité du nombre de pics du premier signal électrocardiographique est inférieure à un second seuil, déterminer (S2034), par le module fonctionnel dans le dispositif électronique (100), que le premier signal électrocardiographique est un signal normal ;

le premier seuil représentant un point de division anormal de la caractéristique de variabilité des pics, ce premier seuil étant déterminé sur la base des caractéristiques de variabilité des pics d'une pluralité d'échantillons de signaux électrocardiographiques,

le second seuil représentant un point de division anormal de la caractéristique de variabilité du nombre de pics, et le second seuil étant déterminé sur la base des caractéristiques de variabilité du nombre de pics de la pluralité d'échantillons de signaux électrocardiographiques.

2. Procédé selon la revendication 1, dans lequel l'obtention d'un premier signal électrocardiographique comprend :

l'obtention d'un signal électrocardiographique original avec une première durée ; et

le traitement de filtrage du signal électrocardiographique original pour obtenir le premier signal électrocardiographique.

3. Procédé selon la revendication 2, dans lequel le traitement de filtrage du signal électrocardiographique original comprend :

le traitement de filtrage passe-bas et le traitement de filtrage passe-haut du signal électrocardiographique original.

4. Le procédé selon l'une quelconque des revendications 1 à 3, dans lequel le calcul d'une caractéristique de variabilité des pics et d'une caractéristique de variabilité du nombre de pics du premier signal électrocardiogramme comprend :

effectuer un traitement de segmentation sur le premier signal électrocardiogramme afin d'obtenir une pluralité de segments de signal ;

calculer une liste de pics et un nombre de pics pour chaque segment de signal, la liste de pics comprenant N pics, et N représentant le nombre de pics ;

obtenir la caractéristique de variabilité des pics du premier signal électrocardiogramme sur la base des listes de pics des différents segments de signal ; et

obtenir la caractéristique de variabilité du nombre de pics du premier signal électrocardiogramme sur la base des nombres de pics des différents segments de signal.

5. Le procédé selon la revendication 4, dans lequel le segment de signal comprend une pluralité de points de données discrets, et le calcul d'une liste de pics pour chaque segment de signal comprend :

calculer une valeur absolue de la différence entre chaque paire de points de données adjacents dans un premier segment de signal, le premier segment de signal étant l'un des segments de signal, une liste de pics du premier segment de signal comprenant un ou plusieurs premiers pics, le premier pic étant une valeur absolue d'une différence supérieure à la valeur absolue de la différence précédente, supérieure à la valeur absolue de la différence suivante et supérieure au seuil de recherche de pic, le seuil de recherche de pic étant déterminé sur la base des valeurs absolues des différences correspondant respectivement à la pluralité de segments de signal du premier électrocardiogramme.

6. Le procédé selon la revendication 5, dans lequel le mode de détermination du seuil de recherche de pic comprend :

calculer un premier produit d'une première constante et d'un premier percentile dans un ensemble des valeurs absolues des différences correspondant à la pluralité de segments de signal du premier électrocardiogramme ;

calculer un second produit d'une seconde constante et d'un second percentile dans l'ensemble des valeurs absolues des différences correspondant à la pluralité de segments de signal du premier électrocardiogramme ; et calculer la différence entre le premier produit et le second produit afin d'obtenir le seuil de recherche de pic.

7. Le procédé selon l'une quelconque des revendications 4 à 6, dans lequel l'obtention de la caractéristique de variabilité des pics du premier signal électrocardiogramme sur la base des listes de pics des différents segments de signal comprend :

calculer l'écart type et la valeur moyenne des pics des différents segments de signal du premier électrocardiogramme ; et calculer le rapport de l'écart-type à la valeur moyenne des pics des différents segments de signal afin d'obtenir la caractéristique de variabilité des pics du premier signal électrocardiogramme.

8. Procédé selon l'une quelconque des revendications 4 à 6, dans lequel l'obtention de la caractéristique de variabilité du nombre de pics du premier signal électrocardiogramme basée sur le nombre de pics des différents segments de signal comprend :

calculer une première différence entre la valeur la plus grande et la valeur la plus petite du nombre de pics des différents segments de signal du premier signal électrocardiogramme ; calculer la valeur médiane du nombre de pics des différents segments de signal du premier signal électrocardiogramme ; et calculer le rapport entre la première différence et la valeur médiane afin d'obtenir la caractéristique de variabilité du nombre de pics du premier signal électrocardiogramme.

9. Procédé selon l'une quelconque des revendications 1 à 8, dans lequel une manière de déterminer le premier seuil et le second seuil comprend :

obtenir plusieurs échantillons de signal électrocardiogramme, chaque échantillon ayant une durée correspondant à la première durée ; calculer une caractéristique de variabilité de pic et une caractéristique de variabilité du nombre de pics pour chaque échantillon de signal électrocardiogramme ; obtenir le premier seuil sur la base des caractéristiques de variabilité de pics des différents échantillons de signal électrocardiogramme ; et obtenir le second seuil sur la base des caractéristiques de variabilité du nombre de pics des différents échantillons de signal électrocardiogramme.

10. Procédé selon la revendication 9, où l'obtention du premier seuil sur la base des caractéristiques de variabilité de pics des différents échantillons de signal électrocardiogramme comprend :

calculer un troisième produit d'une troisième constante et d'un troisième percentile dans les caractéristiques de variabilité de pics des différents échantillons de signal électrocardiogramme ; calculer un quatrième produit d'une quatrième constante et d'un quatrième percentile dans les caractéristiques de variabilité de pics des différents échantillons de signal électrocardiogramme ; et calculer une différence entre le troisième produit et le quatrième produit afin d'obtenir le premier seuil.

11. Procédé selon la revendication 9, où l'obtention du second seuil sur la base des caractéristiques de variabilité du nombre de pics des différents échantillons de signal électrocardiogramme comprend :

calculer un cinquième produit d'une cinquième constante et d'un cinquième percentile dans les caractéristiques de variabilité du nombre de pics des différents échantillons de signal électrocardiogramme ; calculer un sixième produit d'une sixième constante et d'un sixième percentile dans les caractéristiques de variabilité du nombre de pics parmi la pluralité d'échantillons de signaux électrocardiogramme ; et calculer une différence entre le cinquième produit et le sixième produit afin d'obtenir le deuxième seuil.

12. Le procédé selon l'une quelconque des revendications 1 à 11, comprenant en outre :

si la caractéristique de variabilité de pic du premier signal électrocardiogramme est supérieure au premier seuil, et si la caractéristique de variabilité du nombre de pics du premier signal électrocardiogramme est supérieure au deuxième seuil, déterminer (S2031) que le premier signal électrocardiogramme est un signal anormal ;

si la caractéristique de variabilité de pic du premier signal électrocardiogramme est supérieure ou égale au premier seuil, et si la caractéristique de variabilité du nombre de pics du premier signal électrocardiogramme est inférieure ou égale au deuxième seuil, déterminer (S2032) que le premier signal électrocardiogramme est un signal de bruit ; ou

si la caractéristique de variabilité de pic du premier signal électrocardiogramme est inférieure ou égale au premier seuil, et si la caractéristique de variabilité du nombre de pics du premier signal électrocardiogramme est supérieure ou égale au deuxième seuil, déterminer (S2033) que le premier signal électrocardiogramme est un signal de bruit.

13. Le procédé selon l'une quelconque des revendications 9 à 12, dans lequel l'obtention de la pluralité d'échantillons de signaux électrocardiogramme comprend :

obtenir une pluralité de signaux électrocardiogramme originaux ;

effectuer une segmentation des données sur la pluralité de signaux électrocardiogramme originaux afin d'obtenir une pluralité de signaux électrocardiogramme originaux d'une première durée ; et

effectuer un traitement de filtrage passe-bas et passe-haut sur les signaux électrocardiogramme originaux de la première durée pour obtenir la pluralité d'échantillons de signaux électrocardiogramme.

14. Le procédé selon l'une quelconque des revendications 9 à 12, dans lequel la méthode de calcul de la caractéristique de variabilité de pic de chaque échantillon de signal électrocardiogramme est identique à la méthode de calcul de la caractéristique de variabilité de pic du premier signal électrocardiogramme ; et

la méthode de calcul de la caractéristique de variabilité du nombre de pics de chaque échantillon de signal électrocardiogramme est identique à la méthode de calcul de la caractéristique de variabilité du nombre de pics du premier signal électrocardiogramme.

15. Un dispositif électronique (100), comprenant un processeur (110), le processeur étant configuré pour exécuter un programme informatique stocké dans une mémoire (121), afin d'amener le dispositif électronique à mettre en œuvre le procédé selon l'une quelconque des revendications 1 à 14.

Electronic device 100

FIG. 1

| A1<br>Signal<br>collection | → | A2<br>Signal<br>segmentation | → | A3<br>Signal noise<br>reduction | → | A4<br>Feature<br>construction | → | A5<br>Threshold<br>generation |
|---|---|---|---|---|---|---|---|---|

Load
Electronic device

| B1<br>Signal<br>collection | → | B2<br>Signal<br>segmentation | → | B3<br>Signal noise<br>reduction | → | B4<br>Feature<br>construction | → | B5<br>Threshold<br>comparison |
|---|---|---|---|---|---|---|---|---|

| Normal<br>signal | Abnormal<br>signal | Noise<br>signal |
|---|---|---|

FIG. 2

S101: Obtain a plurality of electrocardiogram signal samples

A1
Signal
collection

A2
Signal
segmentation

A3
Signal noise
reduction

S102: Calculate a peak variability feature and a peak number variability feature of each electrocardiogram signal sample

A4
Feature
construction

S103: Obtain the first threshold based on peak variability features of the plurality of electrocardiogram signal samples; and obtain the second threshold based on peak number variability features of the plurality of electrocardiogram signal samples

A5
Threshold
generation

FIG. 3

FIG. 4

FIG. 5

FIG. 6

FIG. 7

FIG. 8

FIG. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2012016249 A1

- US 5842997 A